# EUROPEAN PATENT APPLICATION

(11) **EP 4 438 084 A1**
(43) Date of publication of application: **02.10.2024**
(21) Application number: 22895743.7
(22) Date of filing: 22.11.2022
(51) Int. Cl.: A61M 5/32

(54) **NEEDLE TIP PROTECTOR**

(30) Priority: 22.11.2021 JP 2021189186; 30.05.2022 JP 2022087452
(71) Applicant: Nipro Corporation, Settsu-shi, Osaka 566-8510 (JP)
(72) Inventor: SAKAMOTO, Shingo, Osaka-shi, Osaka 531-8510 (JP)
(74) Representative: Slingsby Partners LLP
(86) International application number: PCT/JP2022/043255
(87) International publication number: WO 2023/090462

(57) **Abstract**

The needle tip protector 10 is attached to a puncture needle 26 and prevents exposure of a needle tip 30 by being moved to the needle tip 30 side, and includes a first side plate 42 and a second side plate 44 extending from a bottom part 40 located on a needle base side of the puncture needle 26 to the needle tip 30 side, and facing each other while sandwiching the puncture needle 26 from both sides. The first side plate 42 and the second side plate 44 include lateral cover pieces 62, 80 protruding inward in a direction of facing at respective widthwise edges, which are opposite to each other in a plate width direction. With the needle tip protector 10 attached to the puncture needle 26, the puncture needle 26 is located between the respective lateral cover pieces 62, 80 of the first side plate 42 and the second side plate 44.

## Description

### TECHNICAL FIELD

This invention relates to a needle tip protector that protects the needle tip of a puncture needle after use.

### BACKGROUND ART

Conventionally, needle tip protectors have been known to be attached to a puncture needle to protect the needle tip of the puncture needle after use by moving the needle tip toward the tip side. As shown, for example, in U.S. Patent No. 4,929,241 (Patent Document 1), the needle tip protector has a set of side plates extending to the needle tip side from the bottom part where the insertion hole for the puncture needle is provided, and the distal end portions of the set of side plates approach each other to form a needle-tip guarding part covering the needle tip.

### BACKGROUND ART DOCUMENT

### PATENT DOCUMENT

Patent Document 1: U.S. Patent No. 4,929,241

### SUMMARY OF THE INVENTION

### PROBLEM THE INVENTION ATTEMPTS TO SOLVE

By the way, it is necessary for needle tip protectors to suppress lateral positioning deviation with respect to puncture needles. For example, when the needle tip protector is in the attached state to the puncture needle or during the withdrawal operation of the puncture needle, by suppressing the lateral positioning deviation of the needle tip protector with respect to the puncture needle, it is possible to maintain a state in which a set of side plates of the needle tip protector extend approximately parallel to the puncture needle, thereby stabilizing the withdrawal operation of the puncture needle. After withdrawal of the puncture needle, re-exposure of the needle tip of the puncture needle housed in the needle tip protector can be prevented by suppressing the lateral positioning deviation of the needle tip protector with respect to the puncture needle.

To suppress lateral positioning deviation of the needle tip protector with respect to the puncture needle after withdrawal of the puncture needle, it is conceivable to provide a pair of side walls at widthwise both edges on one side plate of the needle tip protector, and to house the puncture needle between such pair of side walls. However, the outer diameters of puncture needles vary, and there is a concern that when the puncture needle is thin, the puncture needle and the side walls will be remote from each other and the needle tip may be exposed to the outside due to rattling.

In addition, in the conventionally structured needle tip protector, for example, when the needle tip is covered by the needle-tip cover part on one side plate side and the needle tip tries to move along the needle-tip cover part to the other side plate side, there is a risk that the needle tip may push the other side plate part open and expose itself.

It is therefore one object of the present invention to provide a needle tip protector of novel structure which is able to prevent re-exposure of the needle tip by preventing at least one of lateral positioning deviation of the protector with respect to the puncture needle and push-opening of the protector by the needle tip.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a needle tip protector configured to prevent exposure of a needle tip by being attached to a puncture needle and moved to a needle tip side, comprising: a bottom part located on a needle base side of the puncture needle; a first side plate and a second side plate extending from the bottom part to the needle tip side of the puncture needle, the first side plate and the second side plate being arranged facing each other while sandwiching the puncture needle from both sides; and lateral cover pieces provided to respective widthwise edges of the first side plate and the second side plate and protruding inward in a direction of facing, the respective widthwise edges being opposite to each other in a plate width direction, wherein with the needle tip protector attached to the puncture needle, the puncture needle is located between the respective lateral cover pieces of the first side plate and the second side plate.

According to the needle-tip protector structured following the present preferred embodiment, a single lateral cover piece is formed on each of the first side plate and the second side plate, and those lateral cover pieces are provided on the respective widthwise edges being opposite to each other in the plate width direction of the side plates. Therefore, the distance between the two lateral cover pieces can be set small to stabilize the needle-tip protection by suppressing the movement of the needle-tip, while the first and second side plates having the respective lateral cover pieces can be easily formed.

A second preferred embodiment provides the needle tip protector according to the first preferred embodiment, wherein respective centers of the first side plate and the second side plate in the plate width direction are mutually shifted to sides opposite to the corresponding lateral cover pieces.

According to the needle-tip protector structured following the present preferred embodiment, each center of the first and second side plates in the plate width direction is set to be shifted to the opposite side of the corresponding lateral cover piece. This makes it possible to reliably obtain the width dimension, strength and the like of the first and second side plates, while forming the lateral cover piece sufficiently close to the puncture needle. As a result, for example, deformation of the needle tip protector against external force acted by contact with the puncture needle is suppressed, and the lateral movement of the puncture needle is reliably regulated between both lateral cover pieces to achieve improved stability and reliability of the needle-tip protection state.

A third preferred embodiment provides the needle tip protector according to the first or second preferred embodiment, wherein protrusion pieces are provided to respective other widthwise edges of the first side plate and the second side plate and protrude inward in the direction of facing, the respective other widthwise edges being located opposite to the corresponding lateral cover pieces in the plate width direction, and the protrusion pieces are located on outer sides of the lateral cover pieces.

If the respective other widthwise edges of the first and second side plates being located opposite to the lateral cover pieces spread in a flat plate shape, there is a risk that the needle-tip protection state may be easily released by pushing the said other widthwise edge with a finger or the like. According to the needle-tip protector structured following the present preferred embodiment, the protrusion pieces are provided to the respective other widthwise edges of the first side plate and the second side plate being located opposite to the lateral cover pieces. This makes it difficult to push the other widthwise edges of the first and second lateral cover pieces with a finger or the like, thereby reducing the risk of the needle-tip protection state being released.

A fourth preferred embodiment provides the needle tip protector according to any one of the first through third preferred embodiments, wherein the bottom part has a plate shape including an insertion hole for the puncture needle, and the first side plate and the second side plate are integrally formed with the bottom part such that the first side plate and the second side plate rise from respective edges of the bottom part, and the lateral cover pieces are provided not less than 2.5 mm apart from proximal ends of the first side plate and the second side plate such that a molding space opened to both sides in the plate width direction is provided between facing surfaces of proximal end portions of the first side plate and the second side plate ahead of the bottom part.

According to the needle tip protector structured following the present preferred embodiment, by providing the molding space, it is possible to support the bottom part by a jig (a die) inserted into the molding space and to efficiently perform press working such as raising the first side plate and the second side plate, thereby improving productivity of the needle tip protector, or the like.

A fifth preferred embodiment provides the needle tip protector according to any one of the first through fourth preferred embodiments, wherein in a needle-tip protection state where the first side plate and the second side plate approach each other and the exposure of the needle tip is prevented, protruding distal ends of the lateral cover pieces of the first side plate and the second side plate are respectively pressed against the second side plate and the first side plate.

According to the needle-tip protector structured following the present preferred embodiment, in the needle-tip protection state where the first side plate and the second side plate approach each other, the force required to displace the first side plate and the second side plate in the direction of separation from each other is greater, so that the needle-tip protection state is unlikely to be released.

A sixth preferred embodiment provides a needle tip protector configured to prevent exposure of a needle tip by being attached to a puncture needle and moved to a needle tip side, comprising: a bottom part located on a needle base side of the puncture needle; and a first side plate and a second side plate extending from the bottom part to the needle tip side of the puncture needle, the first side plate and the second side plate being arranged facing each other while sandwiching the puncture needle from both sides, wherein at least one of the first side plate and the second side plate is configured such that a center in a plate width direction is shifted from a needle axis of the puncture needle in the plate width direction, and includes a lateral cover piece at a widthwise edge on a side approaching the puncture needle, the lateral cover piece protruding inward in a direction of facing of the first side plate and the second side plate.

According to the needle-tip protector structured following the present preferred embodiment, the lateral cover piece can be positioned close to the lateral side of the puncture needle while reliably obtain the plate width dimension of the first side plate and/or the second side plate. Thus, the deformation rigidity of the needle-tip protector and the displacement regulation of the needle tip of the puncture needle by the lateral cover piece are achieved in a compatible manner.

A seventh preferred embodiment provides a needle tip protector configured to prevent exposure of a needle tip by being attached to a puncture needle and moved to a needle tip side, comprising: a bottom part located on a needle base side of the puncture needle; and a first side plate and a second side plate extending from the bottom part to the needle tip side of the puncture needle, wherein at least one of the first side plate and the second side plate includes: a notch-shaped concave part opening at a side end in a plate width direction; and a lateral cover piece protruding inward from the notch-shaped concave part and being located on a lateral side of the puncture needle.

According to the needle-tip protector structured following the present preferred embodiment, the lateral cover piece is provided in the concave part formed on the side end of at least one of the first and second side plates. This enables the lateral cover piece to be positioned close to the center in the plate width direction of the side plate, thereby facilitating placement of the lateral cover piece close to the puncture needle. Therefore, the lateral cover piece effectively prevents the needle tip protector from being shifted to the lateral side with respect to the puncture needle.

The width dimension of the needle tip protector on the distal end side with respect to the concave part can be largely obtained, which ensures the necessary deformation rigidity of the needle tip protector on the distal end side. Accordingly, for example, the needle-tip protection state is unlikely to be released easily, thereby realizing stable needle-tip protection for the puncture needle.

At the formation zone of the concave part, where the width dimension is small, the lateral cover piece is provided. This makes it possible to exhibit a reinforcing effect by the lateral cover piece, thereby suppressing a localized decrease in strength of the needle-tip protector.

An eighth preferred embodiment provides a needle tip protector configured to prevent exposure of a needle tip by being attached to a puncture needle and moved to a needle tip side, comprising: a bottom part located on a needle base side of the puncture needle; and a first side plate and a second side plate extending from the bottom part to the needle tip side of the puncture needle, wherein a distal end portion of at least one of the first side plate and the second side plate includes a needle-tip cover part protruding toward the puncture needle, and a protruding distal end of the needle-tip cover part includes a needle-tip stopper part protruding to the needle base side.

In the conventionally structured needle tip protector, for example, when the needle tip is covered by the needle-tip cover part on one side plate side and the needle tip tries to move along the needle-tip cover part to the other side plate side, there is a risk that the needle tip may push the other side plate open and expose itself. According to the needle tip protector structured following the present preferred embodiment, when the needle tip tries to move to the other side plate side as described above, the needle tip is caught by the needle-tip stopper part provided to the needle-tip cover part on the one side plate side and movement to the other side plate side is restricted, thereby preventing the needle tip from coming off the needle-tip cover part and being exposed.

A ninth preferred embodiment provides a needle tip protector configured to prevent exposure of a needle tip by being attached to a puncture needle and moved to a needle tip side, comprising: a bottom part located on a needle base side of the puncture needle; a first side plate and a second side plate extending from the bottom part to the needle tip side of the puncture needle, the first side plate and the second side plate being arranged facing each other while sandwiching the puncture needle from both sides; and a lateral regulating piece extending between facing surfaces of the first side plate and the second side plate, wherein the lateral regulating piece includes a regulating protrusion protruding toward the puncture needle.

According to the needle tip protector of the present preferred embodiment, tilting of the puncture needle is limited by the regulating protrusion of the lateral regulating piece coming into contact with the side surface of the puncture needle, thereby making it possible to prevent the needle tip from being exposed to the lateral side off the needle tip protection part. In particular, the regulating protrusion protrudes from the lateral regulating piece, which is located between the facing surfaces of the first side plate and the second side plate, in the direction approaching the puncture needle. Thus, the protruding distal end of the regulating protrusion can be brought closer to the puncture needle, and the tilting of the puncture needle can be quickly regulated at the stage where the inclination angle is still small.

A tenth preferred embodiment provides the needle tip protector according to the ninth preferred embodiment, wherein the regulating protrusion is provided at an end on the needle tip side of the lateral regulating piece.

According to the needle tip protector of the present preferred embodiment, the regulating protrusion can easily function as a wall to prevent the needle tip from being exposed to the outside, thereby reducing the situation where the needle tip is exposed to the outside. In addition, the regulating protrusion comes into contact with the puncture needle at a position close to the needle tip whose amount of lateral movement increases during tilting of the puncture needle, thereby preventing the puncture needle from tilting in the initial stage of tilting where the inclination angle of the puncture needle is still small.

An eleventh preferred embodiment provides the needle tip protector according to the ninth or tenth preferred embodiment, wherein the lateral regulating piece protrudes inward from the first side plate or the second side plate in a direction of facing of the first side plate and the second side plate.

According to the needle tip protector of the present preferred embodiment, compared to the case where the lateral regulating piece is extended from the bottom part, for example, the lateral regulating piece can be extended to a position closer to the needle tip with a relatively small extension length. This makes it possible to simplify manufacturing and reduce the rate of defective products.

A twelfth preferred embodiment provides a press-formed needle tip protector configured to prevent exposure of a needle tip by being attached to a puncture needle and moved to a needle tip side, comprising: a bottom part located on a needle base side of the puncture needle; and a first side plate and a second side plate bent (by bending process) and extending from the bottom part to the needle tip side of the puncture needle, the first side plate and the second side plate being arranged facing each other, wherein the bottom part has a plate shape including an insertion hole for the puncture needle, and the first side plate and the second side plate are integrally formed with the bottom part such that the first side plate and the second side plate rise from respective edges of the bottom part, and the bottom part includes a convex part protruding outward in a plate width direction of the first side plate and the second side plate.

According to the needle tip protector of the present preferred embodiment, when the needle tip protector is cut out from the blank metal plate by a press to form the needle tip protector, the cut surfaces of the portion other than the convex part can be made into a smooth shape with less burrs, etc. That is, with the portion other than the convex part cut off, bending process is performed to form the first and second side plates, the lateral cover piece, and the like, and then the convex part is cut off to obtain the needle-tip protector. By so doing, it is possible to make it difficult for the cut surfaces other than the convex part to be affected by distortion or the like due to the bending process. As a result, the shape and dimensional accuracy of the cut surfaces other than the convex part can be improved. Besides, a smooth shape can be obtained with less burrs, etc., thereby simplifying the subsequent deburring process. Further, if the cut surfaces other than the convex part, whose dimensions and shapes are highly accurate, are gripped by a jig or the like, high-accuracy positioning becomes possible, and the processing accuracy in the positioned state and the like can be enhanced.

Moreover, for example, when the proximal end of the needle tip protector touches the inner circumferential surface of the needle hub that houses the needle tip protector, the convex part protruding radially outward is likely to touch the needle hub. Thus, by performing the post-processing, such as chamfering, only on the convex part, it is possible to prevent damage or the like to the inner circumferential surface of the needle hub due to touch by the proximal end of the needle tip protector. Therefore, the post-processing work, such as chamfering, can be easier than when the post-processing is performed on the entire widthwise end surface of the bottom part.

A thirteenth preferred embodiment provides a needle tip protector configured to prevent exposure of a needle tip by being attached to a puncture needle and moved to a needle tip side, comprising: a bottom part located on a needle base side of the puncture needle; and a first side plate and a second side plate extending from the bottom part to the needle tip side of the puncture needle, the first side plate and the second side plate being arranged facing each other while sandwiching the puncture needle from both sides, and distal end portions of the first side plate and the second side plate each forming a needle-tip guarding part configured to cover the needle tip by approaching each other, wherein the needle-tip guarding part protrudes outward at a proximal end that is the needle base side, and the proximal end of the needle-tip guarding part includes notch-shaped relief parts on respective sides in a plate width direction.

According to the needle tip protector of the present preferred embodiment, the outer shape of the proximal end of the needle-tip guarding part viewed in the axial direction is curved by the formation of the relief part. Thus, when the needle tip protector is inserted into the inside of the needle hub, etc. from the proximal end side, it is possible to inhibit the inner surface of the needle hub, etc. from being damaged by the proximal end of the needle-tip guarding part, which is the distal end portion of the needle tip protector. That is, when the puncture needle is withdrawn, the relative movement of the needle tip protector and the puncture needle is realized by the proximal end of the needle-tip guarding part coming into contact with the inner surface of the needle hub, etc. However, if the outer shape line of the proximal end of the needle-tip guarding part viewed in the axial projection has a sharp angle, there is a risk that the proximal end of the needle-tip guarding part may scrape the inner surface of the needle hub. Therefore, by providing the notch-shaped relief part at the proximal end of the needle-tip guarding part and making the said part curved when viewed in the axial projection, the risk of scraping the inner surface of the needle hub during contact with the needle hub can be reduced.

A fourteenth preferred embodiment provides a needle tip protector configured to prevent exposure of a needle tip by being attached to a puncture needle and moved to a needle tip side, comprising: a bottom part located on a needle base side of the puncture needle; a first side plate and a second side plate extending from the bottom part to the needle tip side of the puncture needle, the first side plate and the second side plate being arranged facing each other, wherein the bottom part has a plate shape including an insertion hole for the puncture needle, and the first side plate and the second side plate are integrally formed with the bottom part such that the first side plate and the second side plate rise from respective edges of the bottom part, and the bottom part includes a thin-walled stress transmission suppressing part surrounding the insertion hole.

The insertion hole formed in the bottom part requires high dimensional accuracy to engage with, for example, a partial large-diameter part formed near the needle tip of the puncture needle. Here, there is a risk that the insertion hole may be deformed due to input during the bending process performed on the needle tip protector, for example. Therefore, in the present preferred embodiment, by providing the thin-walled part surrounding the insertion hole, the force acting radially inward from the radial outside of the thin-walled part will be absorbed by the deformation of the thin-walled part and be unlikely to be transmitted to the periphery of the insertion hole located on the radial inside of the thin-walled part. Therefore, the insertion hole is unlikely to be deformed due to the action of external force, and the dimension and shape of the insertion hole can be set with high accuracy.

### EFFECT OF THE INVENTION

According to the present invention, the needle tip protector is able to prevent re-exposure of the needle tip by preventing at least one of lateral positioning deviation of the protector with respect to the puncture needle and push-opening of the protector by the needle tip.

### BRIEF DESCRIPTION OF THE DRAWINGS

FIG. 1 is a right side view of a needle assembly including a needle tip protector according to a first practical embodiment of the present invention.
FIG. 2 is a cross sectional view taken along line 2-2 of FIG. 1.
FIG. 3 is a cross sectional view taken along line 3-3 of FIG. 2.
FIG. 4 is an enlarged cross sectional view taken along line 4-4 of FIG. 2.
FIG. 5 is an enlarged perspective view of the needle tip protector constituting the needle assembly of FIG. 1.
FIG. 6 is a perspective view showing the needle tip protector of FIG. 5 at another angle.
FIG. 7 is a top plan view of the needle tip protector shown in FIG. 5.
FIG. 8 is a bottom plan view of the needle tip protector shown in FIG. 5.
FIG. 9 is a right side view of the needle tip protector shown in FIG. 5.
FIG. 10 is a left side view of the needle tip protector shown in FIG. 5.
FIG. 11 is a front view of the needle tip protector shown in FIG. 5.
FIG. 12 is a cross sectional view taken along line 12-12 of FIG. 11.
FIG. 13 is a perspective view of the needle tip protector shown in FIG. 5 in an attached state to an inner needle.
FIG. 14 is a perspective view of a needle tip portion showing a needle-tip protection state by the needle tip protector of FIG. 5.
FIG. 15 is a perspective view showing the needle tip portion of FIG. 14 at another angle.
FIG. 16 is a vertical cross sectional view of the needle tip portion shown in FIG. 14, corresponding to a cross section taken along line 16-16 of FIG. 17.
FIG. 17 is a cross sectional view taken along line 17-17 of FIG. 16.
FIG. 18 is an enlarged top plan view of the needle tip protector in the needle-tip protection state shown in FIG. 14.
FIG. 19 is a bottom plan view of the needle tip protector in the needle-tip protection state shown in FIG. 14.
FIG. 20 is a cross sectional view of a needle assembly including a needle tip protector according to a second practical embodiment of the present invention.
FIG. 21 is a perspective view of the needle tip protector constituting the needle assembly of FIG. 20.
FIG. 22 is a perspective view showing the needle tip protector of FIG. 21 at another angle.
FIG. 23 is a cross sectional view of the needle tip protector shown in FIG. 20, corresponding to a cross section taken along line 23-23 of FIG. 24.
FIG. 24 is a cross sectional view taken along line 24-24 of FIG. 23.
FIG. 25 is a cross sectional view of a needle tip portion showing a needle-tip protection state by the needle tip protector of FIG. 21.
FIG. 26 is a cross sectional view of the needle tip portion showing a state in which a needle tip comes off a second needle-tip cover part in the needle-tip protection state of FIG. 25.
FIG. 27 is a cross sectional view showing a part of a needle assembly including a needle tip protector according to a third practical embodiment of the present invention.
FIG. 28 is a perspective view of the needle tip protector constituting the needle assembly of FIG. 27.
FIG. 29 is a perspective view showing the needle tip protector of FIG. 28 at another angle.
FIG. 30 is a left side view of the needle tip protector shown in FIG. 28.
FIG. 31 is a top plan view of the needle tip protector shown in FIG. 28.
FIG. 32 is a front view of the needle tip protector shown in FIG. 28.
FIG. 33 is a rear view of the needle tip protector shown in FIG. 28.
FIG. 34 is a cross sectional view of a needle tip portion showing a needle-tip protection state by the needle tip protector of FIG. 28, corresponding to a cross section taken along line 34-34 of FIG. 35.
FIG. 35 is a cross sectional view taken along line 35-35 of FIG. 33.

### EMBODIMENTS FOR CARRYING OUT THE INVENTION

The practical embodiments of the invention will be described below with reference to the drawings.

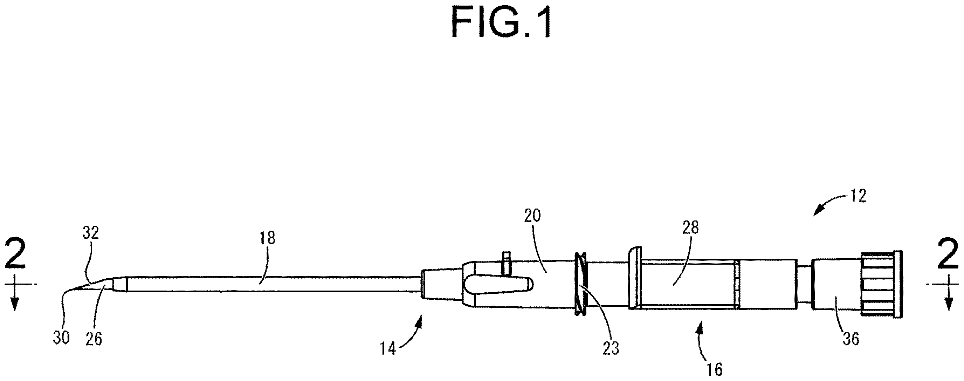

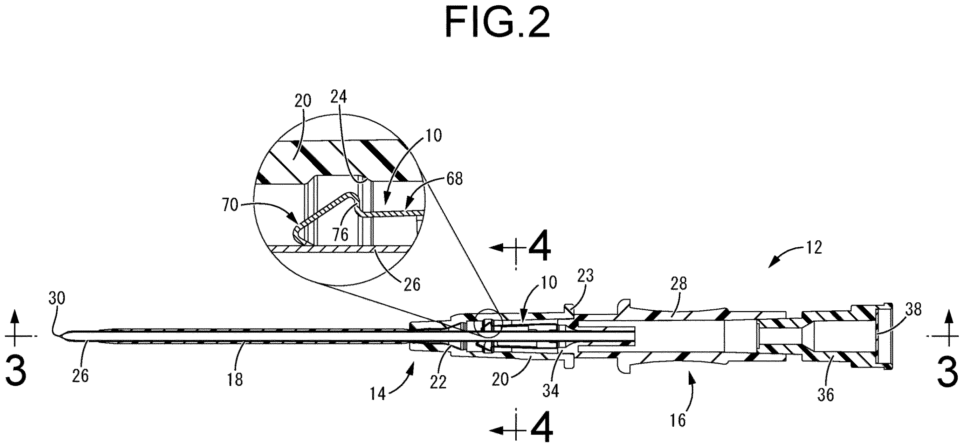

FIGS. 1 to 4 show a needle assembly 12 including a needle tip protector 10 according to a first practical embodiment of the present invention. The needle assembly 12 is constituted by an outer needle unit 14 and an inner needle unit 16 being combined. In the following description, as a general rule, the front-back direction refers to the left-right direction in FIG. 1, which is the needle axis direction. The vertical direction refers to the vertical direction in FIG. 1, and the left-right direction refers to the vertical direction in FIG. 2. Besides, as a general rule, the left side in FIG. 1, which is the side of a needle tip 30 (to be described later), is the distal end, and the right side in FIG. 1 is the proximal end.

The outer needle unit 14 has a structure in which an outer needle hub 20 is fixed to the proximal end portion of an outer needle 18. The outer needle 18 is a tube-shaped member made of synthetic resin or the like, and the outer diameter dimension of the distal end portion decreases toward the distal end. The proximal end portion of the outer needle 18 is inserted into the distal end portion of the tubular outer needle hub 20, and is fixed to the outer needle hub 20 by a caulking pin 22 that is press-fitted into the proximal end portion of the outer needle 18 and the distal end portion of the outer needle hub 20.

The outer needle hub 20 is a member having an approximately round tubular shape overall, and is formed of rigid synthetic resin. The outer needle hub 20 has a luer taper on its inner circumferential surface that increases in diameter toward the proximal end side. An external circuit such as a syringe or infusion line can be connected to the lumen of the outer needle hub 20 by fitting a syringe tip or a connector (not shown) to the lumen of the outer needle hub 20. In addition, a screw part 23 protruding radially outward is formed at the proximal end part of the outer needle hub 20. By the screw part 23 being screwed with a locking part (not shown), it is possible to prevent the syringe, infusion line, or other external circuit from accidentally slipping out of the outer needle hub 20.

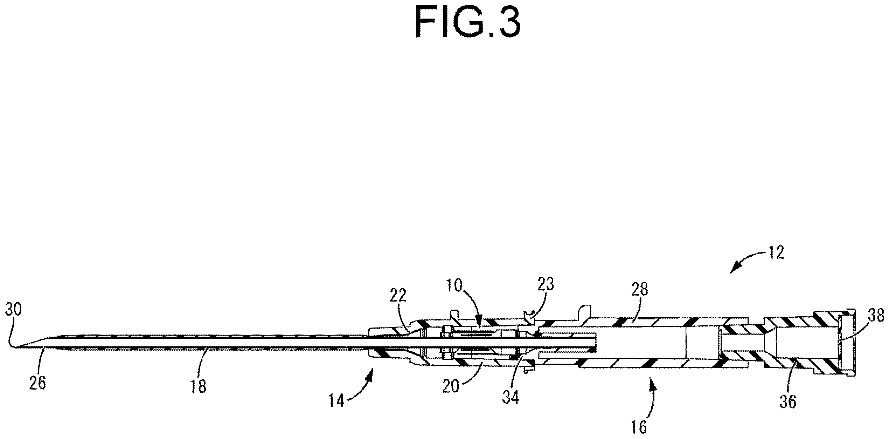

A locking shoulder part 24 is formed on the inner circumferential surface of the outer needle hub 20. The locking shoulder part 24 is a step provided so that the lumen of the outer needle hub 20 is narrower on the proximal end side than on the distal end side. As shown enlarged in FIG. 3, the locking shoulder part 24 of this practical embodiment is constituted by a side wall face on the proximal end side of a concave groove that opens onto the inner circumferential surface of the outer needle hub 20 and extends annularly in the circumferential direction, and is provided continuously over the entire circumference.

The inner needle unit 16 has a structure in which an inner needle hub 28 is attached to the proximal end portion of an inner needle 26 serving as a puncture needle. The inner needle 26 is a hollow needle formed of medical metal such as stainless steel, and has a sharp needle tip 30 at the distal end. The needle tip 30 is formed by the distal end portion of the inner needle 26 having a blade surface 32 that extends so as to incline with respect to the needle axis. The proximal end portion of the inner needle 26 is inserted into the distal end portion of a tubular inner needle hub 28, and those inner needle 26 and inner needle hub 28 are fixed by means of adhesion or the like.

The inner needle hub 28 is a member having an approximately round tubular shape, and is formed of rigid synthetic resin. The distal end part of the inner needle hub 28 includes a cylindrical connecting part 34 that can be fitted into the proximal end opening of the outer needle hub 20. A filter cap 36 is fitted into the proximal end opening of the inner needle hub 28.

The outer needle unit 14 and the inner needle unit 16 form the needle assembly 12 by being mutually combined in the state where the connecting part 34 of the inner needle hub 28 is fitted into the proximal end opening of the outer needle hub 20 and the inner needle 26 is inserted through the outer needle 18 and the outer needle hub 20. By applying a tensile force to the inner needle unit 16 toward the proximal end side with respect to the outer needle unit 14, the fitting of the connecting part 34 to the outer needle hub 20 will be released, so that the outer needle unit 14 and the inner needle unit 16 can be mutually separated.

The needle tip protector 10 is attached to the inner needle 26. The needle tip protector 10 is formed of a metallic material such as medical stainless steel, for example. In this practical embodiment, the parts described below that constitute the needle tip protector 10 are integrally formed, and the needle tip protector 10 can be easily manufactured by, for example, press working of a blank metal plate.

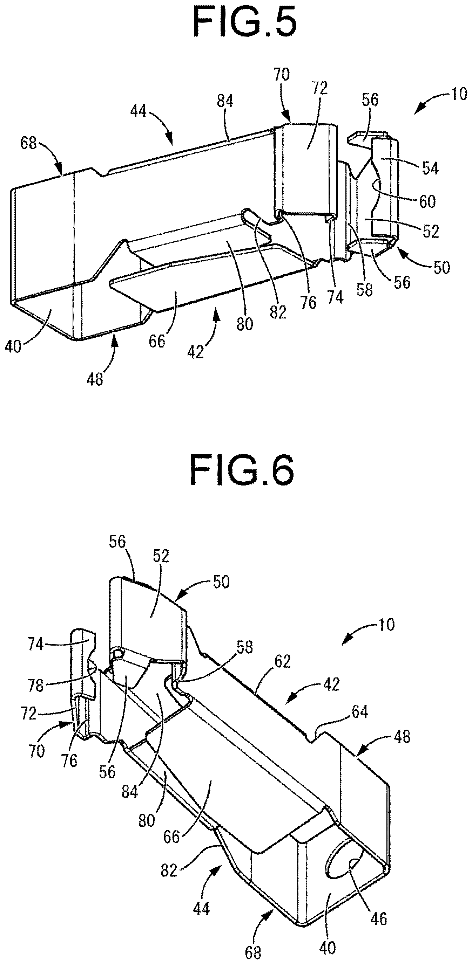

As shown in FIGS. 5 to 12, the needle tip protector 10 has a structure in which a first side plate 42 and a second side plate 44 extend out toward the needle tip 30 side from the both edges of a bottom part 40 located on the needle base side (the opposite side to the needle tip 30) of the inner needle 26. In FIGS. 5 to 12, for illustrative purposes, the needle tip protector 10 is shown in the pre-operating state (before the needle tip is protected), but the needle tip protector 10 may be formed in the post-operating mode (after the needle tip is protected) and made into the pre-operating state shown in FIGS. 5 to 12 by attachment to the inner needle 26.

As shown in FIG. 6, the bottom part 40 has as an approximately rectangular flat-plate shape, and is penetrated by an insertion hole 46 in the plate thickness direction. At a facing pair of edges of the bottom part 40, the first side plate 42 and the second side plate 44 are integrally formed and extend out to one side of the bottom part 40 in the thickness direction. The first side plate 42 and the second side plate 44 of this practical embodiment are arranged facing each other while sandwiching the inner needle 26 from the both sides, and do not extend in an intersecting manner.

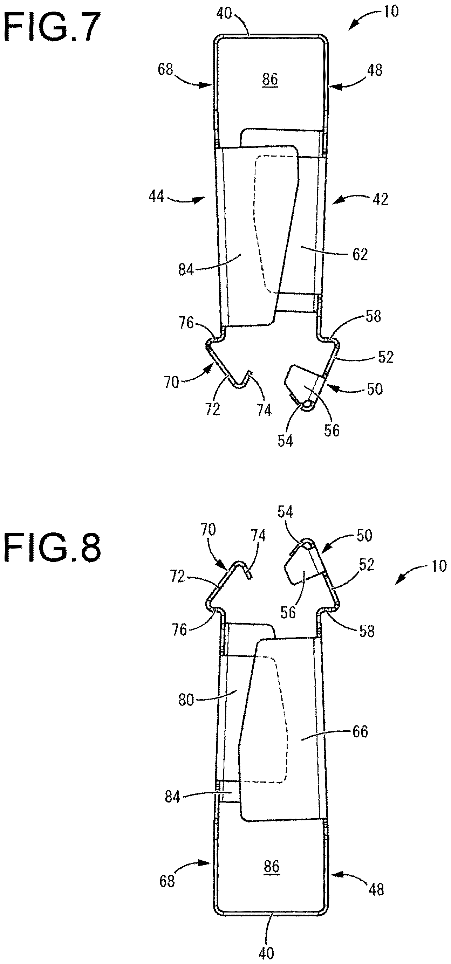

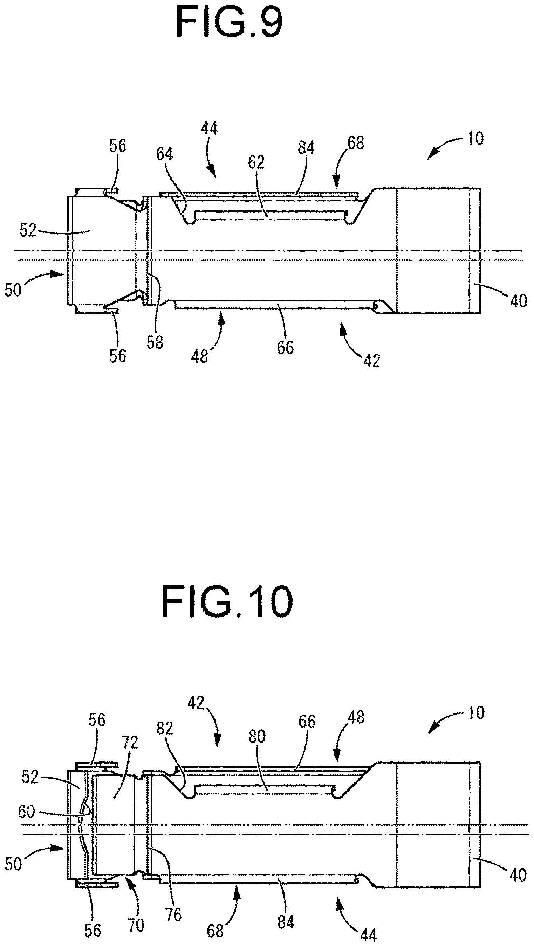

The first side plate 42 has an approximately rectangular plate shape overall, and is elongated in the length direction (the left-right direction in FIG. 7), which is the direction of extension from the bottom part 40. The first side plate 42 includes a first main body 48 extending from the bottom part 40 and a first needle-tip protection part 50 located on the distal end side with respect to the first main body 48. The first side plate 42 suitably has a plate width dimension of 1 to 5 mm. This ensures sufficient deformation rigidity in the first side plate 42 to effectively perform the functions of guiding the withdrawal of the inner needle 26 and protecting the needle tip 30 of the inner needle 26. Also, this prevents the die for bending the blank metal plate (the pressing jig to be described later) from becoming excessively elongated when press-molding the first side plate 42, which will be described later, thereby reliably obtaining durability of the die. In the following description of needle tip protector 10, as a general rule, the length direction refers to the vertical direction in FIG. 7, which is the direction of extension of the first and second side plates 42, 44 from the bottom part 40. Besides, the width direction refers to the vertical direction in FIG. 9, which is the plate width direction of the first and second side plates 42, 44.

The first needle-tip protection part 50 includes a first distal-end protection piece 52 protruding to the distal end side while inclining to the second side plate 44 side. On the distal end side of the first distal-end protection piece 52, provided is a first return piece 54 protruding to the second side plate 44 side while inclining toward the bottom part 40. On both sides in the width direction of the distal end portion of the first distal-end protection piece 52, provided are a pair of lateral guarding pieces 56, 56 extending approximately orthogonally to the width direction and protruding in approximately the same direction as the first return piece 54. The proximal end of the first distal-end protection piece 52 includes a first stepped part 58 protruding outward toward the second side plate 44, and the first stepped part 58 is integrally continuous with the distal end of the first main body 48. In other words, the proximal end of the first distal-end protection piece 52 is shifted by the first stepped part 58 to the opposite side of the second side plate 44 with respect to the distal end of the first main body 48.

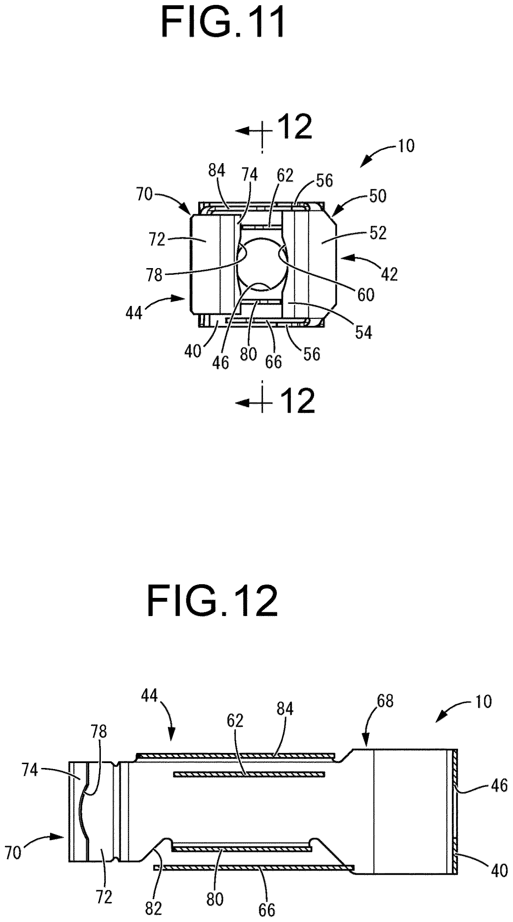

A first notch 60 is formed at the projecting distal end edge of the first return piece 54 from the first distal-end protection piece 52. As shown in FIG. 11, the first notch 60 is provided in the widthwise center portion of the first return piece 54, and has a width dimension that is larger than the wall thickness dimension (the axial dimension) of the first return piece 54 while extending in the width direction with a length shorter than the width dimension of the first return piece 54. The inner surface of the first notch 60 is an approximately arcuate curved surface with a curvature smaller than that of the outer circumferential surface of the inner needle 26, and the depth dimension of the first notch 60 increases toward the widthwise center. The inner surface of the first notch 60 has an arcuate shape less than half a circumference, and the length dimension of the first notch 60 in the width direction is larger than the diameter of the inner needle 26.

The first main body 48 includes a first lateral cover piece 62 serving as a lateral cover piece. As shown in FIGS. 7 and 9, the first lateral cover piece 62 extends from one widthwise edge of the first main body 48 toward the second side plate 44, and is provided at the lengthwise middle position of the first main body 48 so as to be biased toward the distal end side.

That is, a first concave part 64 opens at one widthwise edge of the first main body 48, and the first main body 48 is made narrower at the portion where the first concave part 64 is formed. As shown in FIG. 9, the first concave part 64 has a shape of a notch penetrating the first main body 48 in the plate thickness direction, and is provided in the middle portion of the first main body 48 in the front-back direction, and does not reach both ends of the first main body 48 in the front-back direction. Accordingly, the first main body 48 is made narrower at the portion where the first concave part 64 is formed than the front-back both outer sides of the first concave part 64. The vertical depth dimension of the first concave part 64 at front-back both ends decreases toward the both outer sides in the front-back direction. In other words, the length dimension of the first concave part 64 in the front-back direction increases toward the opening to the lateral side. The depth dimension of the first concave part 64 is the largest on both sides of the first lateral cover piece 62 in the front-back direction.

The first lateral cover piece 62 is provided protruding inward in the left-right direction at the bottom of the first concave part 64, and is located inside in the width direction with respect to the widthwise outer edge of the first main body 48 including the opening edge of the first concave part 64. The protrusion height of the first lateral cover piece 62 from the first side plate 42 is approximately constant at the proximal end portion near the bottom part 40, and gradually decreases toward the distal end at the distal end portion near the first needle-tip protection part 50. The first lateral cover piece 62 suitably has a length dimension of 1 to 5 mm and a maximum protrusion height dimension of 1 to 3 mm from the first main body 48. This ensures, for example, that the first lateral cover piece 62 covers the lateral side of the inner needle 26 after the needle tip 30 is protected as described below, and also avoids malfunctions or the like due to contact between the protruding distal end of the first lateral cover piece 62 and the second side plate 44.

The first main body 48 is provided with a first protrusion piece 66 serving as a protrusion piece. As shown in FIG. 8, the first protrusion piece 66 extends from the other widthwise edge of the first main body 48 toward the second side plate 44, and is provided at the lengthwise middle position of the first main body 48 so as to be biased toward the distal end side. The first protrusion piece 66 is provided at the edge of the first main body 48 opposite to the first lateral cover piece 62, and is remote from and face the first lateral cover piece 62 in the width direction. In this practical embodiment, the first lateral cover piece 62 and the first protrusion piece 66 spread approximately parallel to each other. As shown in FIG. 7, the first protrusion piece 66 has a length dimension (the vertical dimension in FIG. 7) larger than that of the first lateral cover piece 62, and extends to the both outer sides from the first lateral cover piece 62 in the length direction. The protrusion height of the first protrusion piece 66 from the first main body 48 is approximately the same as that of the first lateral cover piece 62. Like the first lateral cover piece 62, the protrusion height of the first protrusion piece 66 is approximately constant at the proximal end portion in the length direction, and gradually decreases toward the distal end at the distal end portion in the length direction.

The center position between the first lateral cover piece 62 and the first protrusion piece 66 in the direction of facing (the width direction), indicated by the dot-and-dash line in FIG. 9, is shifted to the first protrusion piece 66 side in the width direction from the widthwise center position of the bottom part 40, indicated by the chain double-dashed line in FIG. 9. In other words, in the portion of the first main body 48 where the first lateral cover piece 62 and the first protrusion piece 66 are formed, due to the formation of the first concave part 64, the center of the first main body 48 in the plate width direction (the dot-and-dash line in FIG. 9) is shifted from the center in the same direction of the bottom part 40 (the chain double-dashed line in FIG. 9) to the opposite side of the first lateral cover piece 62 in the width direction (to the first protrusion piece 66 side). The center position of the bottom part 40 is approximately coincident with the center axis of the insertion hole 46, which is approximately coincident with the needle axis of the inner needle 26 inserted through the insertion hole 46. Thus, the chain double-dashed line in FIG. 9 indicates the position of the needle axis of the inner needle 26 in the width direction.

On the other hand, the second side plate 44 has an approximately rectangular plate shape overall, and is elongated in the length direction of extension from the bottom part 40 (the vertical direction in FIG. 7). The second side plate 44 includes a second main body 68 extending from the bottom part 40 and a second needle-tip protection part 70 located on the distal end side with respect to the second main body 68. The second side plate 44 suitably has a plate width dimension of 1 to 5 mm.

The second needle-tip protection part 70 includes a second distal-end protection piece 72 that inclines to the first side plate 42 side toward the distal end side. Besides, on the distal end side of the second distal-end protection piece 72, provided is and a second return piece 74 protruding to the first side plate 42 side while inclining toward the bottom part 40. The proximal end of the second distal-end protection piece 72 includes a second stepped part 76 protruding outward toward the first side plate 42, and the second stepped part 76 is integrally continuous with the distal end of the second main body 68 of the second side plate 44. In other words, the proximal end of the second distal-end protection piece 72 is shifted by the second stepped part 76 to the opposite side of the first side plate 42 with respect to the distal end of the second main body 68. The second needle-tip protection part 70 is not provided with a lateral guarding piece corresponding to the lateral guarding pieces 56, 56 of the first needle-tip protection part 50, and the lateral sides of the second needle-tip protection part 70 are open without being covered. That is, the pair of lateral guarding pieces 56, 56 on both sides in the plate width direction are both provided to the first needle-tip protection part 50.

A second notch 78 is formed in the projecting distal end edge of the second return piece 74 from the second distal-end protection piece 72. As shown in FIG. 11, the second notch 78 is provided in the widthwise center portion of the second return piece 74, and has a width dimension that is larger than the wall thickness dimension (the axial dimension) of the second return piece 74 while extending in the width direction with a length shorter than the width dimension of the second return piece 74. The inner surface of the second notch 78 is an approximately arcuate curved surface with a curvature smaller than that of the outer circumferential surface of the inner needle 26, and the depth dimension of the second notch 78 increases toward the widthwise center. The inner surface of the second notch 78 has an arcuate shape less than half a circumference, and the length dimension of the second notch 78 in the width direction is larger than the diameter of the inner needle 26.

The second main body 68 includes a second lateral cover piece 80 serving as a lateral cover piece. As shown in FIGS. 8 and 10, the second lateral cover piece 80 extends from widthwise one end edge of the second main body 68 toward the first side plate 42, and is provided at the lengthwise middle position of the second main body 68 so as to be biased toward the distal end side.

That is, a second concave part 82 opens at one side end of the second main body 68, and the second main body 68 is made narrower at the second concave part 82. As shown in FIG. 10, the second concave part 82 has a shape of a notch penetrating the second main body 68 in the plate thickness direction, and is provided in the middle portion of the second main body 68 in the front-back direction, and does not reach both ends of the second main body 68 in the front-back direction. Accordingly, the second main body 68 is made narrower at the portion where the second concave part 82 is formed than the front-back both outer sides of the second concave part 82. The vertical depth dimension of the second concave part 82 at front-back both ends decreases toward the both outer sides in the front-back direction. In other words, the length dimension of the second concave part 82 in the front-back direction increases toward the opening to the lateral side. The depth dimension of the second concave part 82 is the largest on both sides of the second lateral cover piece 80 in the front-back direction.

The second lateral cover piece 80 is provided protruding inward in the left-right direction at the bottom of the second concave part 82, and is located inside in the width direction with respect to the widthwise outer edge of the second main body 68 including the opening edge of the second concave part 82. The protrusion height of the second lateral cover piece 80 from the second side plate 44 is approximately constant at the proximal end portion near the bottom part 40, and gradually decreases toward the distal end at the distal end portion near the second needle-tip protection part 70. The second lateral cover piece 80 suitably has a length dimension of 1 to 5 mm and a maximum protrusion height of 1 to 3 mm from the second main body 68.

The second main body 68 is provided with a second protrusion piece 84 serving as a protrusion piece. As shown in FIG. 7, the second protrusion piece 84 extends from the other widthwise edge of the second main body 68 toward the first side plate 42, and is provided at the lengthwise middle position of the second main body 68 so as to be biased toward the distal end side. The second protrusion piece 84 is provided at the edge of the second main body 68 opposite to the second lateral cover piece 80, and is remote from and face the second lateral cover piece 80 in the width direction. In this practical embodiment, the second lateral cover piece 80 and the second protrusion piece 84 spread approximately parallel to each other. As shown in FIG. 8, the second protrusion piece 84 has a length dimension (the vertical dimension in FIG. 8) larger than that of the second lateral cover piece 80, and extends to the both outer sides from the second lateral cover piece 80 in the length direction. The protrusion height of the second protrusion piece 84 from the second main body 68 is approximately the same as that of the second lateral cover piece 80. Like the second lateral cover piece 80, the protrusion height of the second protrusion piece 84 is approximately constant at the proximal end portion in the length direction, and gradually decreases toward the distal end at the distal end portion in the length direction.

The center position between the second lateral cover piece 80 and the second protrusion piece 84 in the direction of facing (the width direction), indicated by the dot-and-dash line in FIG. 10, is shifted to the second protrusion piece 84 side in the width direction from the widthwise center position of the bottom part 40, indicated by the chain double-dashed line in FIG. 10. In other words, in the portion of the second main body 68 where the second lateral cover piece 80 and the second protrusion piece 84 are formed, the center of the second main body 68 in the plate width direction (the dot-and-dash line in FIG. 10) is shifted from the center in the same direction of the bottom part 40 (the chain double-dashed line in FIG. 10) to the opposite side of the second lateral cover piece 80 in the width direction (to the second protrusion piece 84 side). Accordingly, the center of the first main body 48 in the plate width direction at the portion where the first lateral cover piece 62 and the first protrusion piece 66 are formed (the dot-and-dash line in FIG. 9) and the center of the second main body 68 in the plate width direction at the portion where the second lateral cover piece 80 and the second protrusion piece 84 are formed (the dot-and-dash line in FIG. 10) are mutually shifted to the sides opposite to the corresponding lateral cover pieces 62, 80. Like the chain double-dashed line in FIG. 9, the chain double-dashed line in FIG. 10 is also approximately coincident with the position of the needle axis of the inner needle 26 in the width direction.

The first lateral cover piece 62 and the second protrusion piece 84 are provided at the same side edge in the width direction in the first side plate 42 and the second side plate 44, and overlap with each other in the width direction in the state of attachment to the inner needle 26 before the needle tip is protected. As shown in FIG. 12, the first lateral cover piece 62 is positioned inwardly apart from the second protrusion piece 84 in the width direction. The first lateral cover piece 62 has a protrusion height that does not reach the second side plate 44, and the second protrusion piece 84 has a protrusion height that does not reach the first side plate 42, and those first lateral cover piece 62 and second protrusion piece 84 partially overlap with each other. As shown in FIG. 7, the second protrusion piece 84 extends to the both outer sides from the first lateral cover piece 62 in the length direction. The lengthwise center position of the second protrusion piece 84 is biased from the lengthwise center position of the first lateral cover piece 62 to the side opposite to the bottom part 40 (the distal end side).

Meanwhile, the second lateral cover piece 80 and the first protrusion piece 66 are provided at the same side edge in the width direction in the first side plate 42 and the second side plate 44, and overlap with each other in the width direction in the state of attachment to the inner needle 26 before the needle tip is protected. As shown in FIG. 12, the second lateral cover piece 80 is positioned inwardly apart from the first protrusion piece 66 in the width direction. The second lateral cover piece 80 has a protrusion height that does not reach the first side plate 42, and the first protrusion piece 66 has a protrusion height that does not reach the second side plate 44, and those second lateral cover piece 80 and first protrusion piece 66 partially overlap with each other. As shown in FIG. 8, the first protrusion piece 66 extends to the both outer sides from the second lateral cover piece 80 in the length direction. The lengthwise center position of the first protrusion piece 66 is biased from the lengthwise center position of the second lateral cover piece 80 to the bottom part 40 side.

The first lateral cover piece 62 and the second lateral cover piece 80 are provided on the sides opposite to each other in the width direction, and are apart from and face each other in the width direction. Besides, the first protrusion piece 66 and the second protrusion piece 84 are provided on the sides opposite to each other in the width direction, and are apart from and face each other in the width direction, while being located on the both outer sides of the first and second lateral cover pieces 62, 80 in the width direction.

In this practical embodiment, the first lateral cover piece 62 is larger in length dimension than the second lateral cover piece 80, and the distal end position of the first lateral cover piece 62 is approximately the same as that of the second lateral cover piece 80, while the proximal end position thereof is closer to the bottom part 40 than that of the second lateral cover piece 80. Meanwhile, the first protrusion piece 66 and the second protrusion piece 84 have approximately the same length dimension, but the first protrusion piece 66 is shifted closer to the bottom part 40 than the second protrusion piece 84.

As shown in FIGS. 7 and 8, the first and second lateral cover pieces 62, 80 and the first and second protrusion pieces 66, 84 are all apart from the bottom part 40 to the distal end side in the length direction. A molding space 86 is formed between the bottom part 40 and the first and second side cover pieces 62, 80 as well as the first and second protruding pieces 66, 84, the molding space 86 penetrating in the width direction and being opened to both sides in the width direction between the facing surfaces of the first and second side plates 42, 44. It is desirable that the first and second lateral cover pieces 62, 80 and the first and second protrusion pieces 66, 84 be formed not less than 2.5 mm apart from the proximal ends of the first and second side plates 42, 44 (the distal end face of the bottom part 40), and more suitably not less than 3.5 mm. That is, the lengthwise dimension of the molding space 86 is suitably not less than 2.5 mm, and more suitably not less than 3.5 mm. Besides, the lengthwise dimension of the molding space 86 is suitably not greater than 7 mm, more suitably not greater than 5 mm. This prevents the die (the pressing jig to be described later) inserted into the molding space 86 from becoming excessively thin-walled in the length direction, thereby obtaining sufficient strength and durability of the die, as well as minimizing the length dimension of the needle tip protector 10, so as to achieve downsizing of the outer needle hub 20, which houses the needle tip protector 10, or the like.

The needle tip protector 10 is formed, for example, by bending a blank metal plate punched into a predetermined shape by press working (progressive press). During the press working, it is possible to process while holding down the bottom part 40 by utilizing the molding space 86. That is, during the press working, the portion corresponding to the bottom part 40 is clasped in the plate thickness direction by a support jig (not shown), which supports one face, and a pressing jig (not shown), which supports the other face. The pressing jig is shaped to be able to be inserted through the molding space 86 and is overlapped with the face of the bottom part 40 from which the first and second side plates 42, 44 rise up. This makes it possible to hold the bottom part 40 in a clasped state from the start to the end of the press working, thereby facilitating molding of the needle tip protector 10. The pressing jig also functions as a die for bending and forming the first and second side plates 42, 44 with respect to the bottom part 40. The blank metal plate to be processed into the needle tip protector 10 by the progressive press is formed, for example, by stainless steel or titanium for medical use, and is suitably a plate material having a thickness of 0.1 to 0.3 mm. By employing a blank metal plate in such a thickness range, it is possible to obtain a needle tip protector 10 with sufficiently low sliding resistance with respect to the inner needle 26 and with deformation rigidity that can stably maintain the needle tip protection mode, and damage or the like during the press working is also unlikely to be a problem. The needle tip protector 10 may have an approximately constant thickness dimension in its entirety, or may partially have thick-walled and/or thin-walled portions.

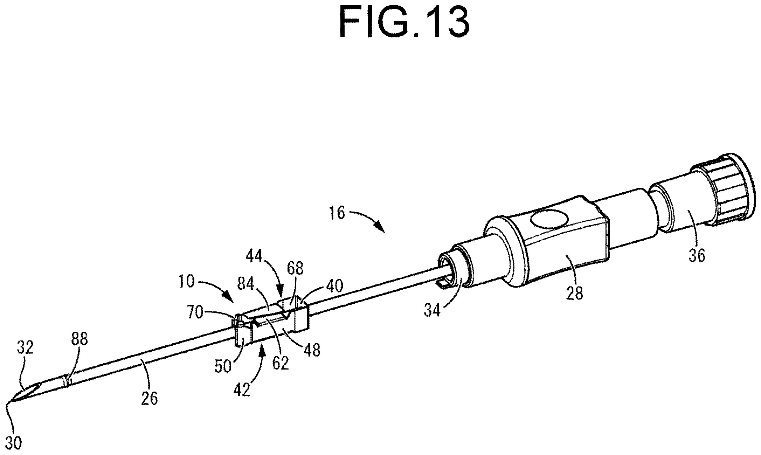

The needle tip protector 10 of the above construction is attached to the inner needle 26, as shown in FIG. 13. That is, the inner needle 26 is inserted through the insertion hole 46 formed in the bottom part 40 of the needle tip protector 10, and is also inserted between the first return piece 54 of the first needle-tip protection part 50 and the second return piece 74 of the second needle-tip protection part 70. With this configuration, the inner needle 26 is arranged to penetrate between the facing surfaces of the first side plate 42 and the second side plate 44 of the needle tip protector 10, and the needle tip protector 10 is externally attached onto the inner needle 26. The inner needle 26 is located between the facing surfaces of the first lateral cover piece 62 and the second lateral cover piece 80 of the needle tip protector 10, and is surrounded in part in the needle axis direction by the first and second side plates 42, 44 and the first and second lateral cover pieces 62, 80. In FIG. 13, for illustrative purposes, the needle tip protector 10 is positioned away from the inner needle hub 28 to the needle tip 30 side. However, as shown in FIGS. 2 and 3, the needle tip protector 10 can be positioned with the bottom part 40 in contact with or close to the distal end of the inner needle hub 28.

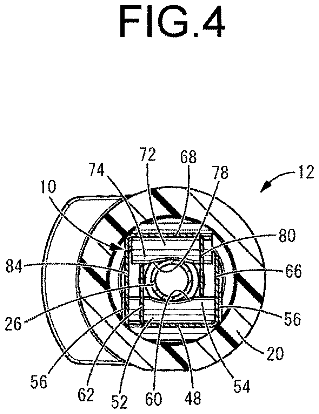

With the needle tip protector 10 attached to the inner needle 26, as shown in FIGS. 2 and 4, the inner needle 26 is inserted between the first notch 60 of the first return piece 54 and the second notch 78 of the second return piece 74, with the inner surfaces of the first and second notches 60, 78 being in contact with the outer circumferential surface of the inner needle 26. By the first and second return pieces 54, 74 being in contact with the inner needle 26 at the first and second notches 60, 78, the first and second side plates 42, 44 are elastically deformed. Due to elastic recovery force of the first and second side plates 42, 44, the first and second return pieces 54, 74 are pressed against the inner needle 26. The inner surfaces of the first and second notches 60, 78 have a curvature smaller than that of the outer circumferential surface of the inner needle 26, so that the contact area between the inner needle 26 and the first and second return pieces 54, 74 at the first and second notches 60, 78 is made small.

With the needle tip protector 10 attached to the inner needle 26, the needle axis of the inner needle 26 is shifted from the center of the first side plate 42 in the plate width direction of the first side plate 42 of the needle tip protector 10 to the side approaching the first lateral cover piece 62 in the plate width direction of the first side plate 42. Besides, the needle axis of the inner needle 26 is shifted from the center of the second side plate 44 in the plate width direction of the second side plate 44 of the needle tip protector 10 to the side approaching the second lateral cover piece 80 in the plate width direction of the second side plate 44. In other words, the first and second side plates 42, 44, which are shifted from the needle axis of the inner needle 26 in the plate width direction, respectively include the first and second lateral cover pieces 62, 80 at the respective widthwise edges on the side approaching the inner needle 26.

The needle tip protector 10 is housed in the lumen of the outer needle hub 20, as shown in FIGS. 2 to 4. The distal end portion of the needle tip protector 10 is tapered by the first and second distal-end protection pieces 52, 72 having inclined shapes that approach each other toward the distal end. This prevents damage to the inner circumferential surface of the outer needle hub 20 or the like due to unintended catching of the needle tip protector 10 when the needle tip protector 10 is inserted into the outer needle hub 20. Widthwise both end portions of the first and second stepped parts 58, 76 of the needle tip protector 10 are axially locked to the locking shoulder part 24 provided to the inner circumferential surface of the outer needle hub 20, whereby movement of the needle tip protector 10 toward the proximal end side with respect to the outer needle hub 20 is limited. In such an attached state of the needle tip protector 10, the outermost rectangular parts of the bottom part 40 and the needle-tip guarding parts 50, 70, for example, can be placed in close proximity or contact with the inner circumferential surface of the outer needle hub 20 in order to suppress unnecessary rattling of the needle tip protector 10 with respect to the inner needle 26. In addition, in order to suppress free rotation of the needle tip protector 10 around the inner needle 26 in such an attached state, the connecting part 34 of the inner needle hub 28 inserted into the outer needle hub 20 may protrude to the distal end side partially in the circumferential direction (see FIGS. 3 and 13), so that the said protruding portion enter the outside of the outer peripheral edge of the bottom part 40 of the needle tip protector 10.

Regarding the needle assembly 12 including such a needle tip protector 10, for example, the inner needle 26 and the outer needle 18 puncture a blood vessel of a patient or the like. Blood flows into the lumen of the inner needle hub 28 through the lumen of the inner needle 26 when puncturing a blood vessel. Thus, for example, by making the inner needle hub 28 transparent or translucent so that the interior can be seen, the puncture of the inner needle 26 into the blood vessel can be confirmed by the inflow of the blood into the inner needle hub 28. Besides, the filter 38 of the filter cap 36 attached to the inner needle hub 28 can prevent the blood that has flowed into the lumen of the inner needle hub 28 from leaking out of the opening at the proximal end side.

After puncturing the blood vessel with the inner needle 26 and the outer needle 18, the inner needle 26 can be withdrawn from the outer needle 18 to the proximal end side by pulling the inner needle unit 16 toward the proximal end side while holding the outer needle unit 14 in the puncture position. At this time, the connection between the inner needle hub 28 and the outer needle hub 20 is released, and the entire inner needle unit 16 is separated from the outer needle unit 14.

During withdrawal of the inner needle 26 to the proximal end side, the edges of the first and second return pieces 54, 74 of the needle tip protector 10 slide against the outer circumferential surface of the inner needle 26. Since the first and second return pieces 54, 74 incline to the proximal end side toward the inner needle 26, the edges of the first and second return pieces 54, 74 are unlikely to get caught on the fine irregularities of the outer circumferential surface of the inner needle 26 during withdrawal of the inner needle 26. Thus, sliding resistance is suppressed to realize a smooth withdrawal of the inner needle 26 toward the proximal end side.

When the inner needle 26 is withdrawn to the proximal end side, the inner needle 26 is guided in the axial direction by the first and second notches 60, 78 provided to the first and second return pieces 54, 74 of the needle tip protector 10 to assist in smooth withdrawal of the inner needle 26 to the proximal end side. The length dimension of the first and second notches 60, 78 in the width direction (the left-right direction in FIG. 4) is larger than the diameter of the inner needle 26. Thus, even if the inner needle 26 moves slightly in the width direction, the inner needle 26 unlikely to come off the first and second notches 60, 78, thereby effectively obtaining the guiding function of the inner needle 26 by the first and second notches 60, 78.

Since the first and second notches 60, 78 are formed extending in the width direction of the first and second return pieces 54, 74, the inner needle 26 and the first and second return pieces 54, 74 are in contact within a narrow range in the needle axis direction. Thus, for example, the sliding resistance due to surface roughness of the contact portion is reduced, thereby facilitating the withdrawal operation of the inner needle 26. The thickness dimension of the first and second return pieces 54, 74 is sufficiently small compared to the outer diameter of the inner needle 26. Especially in this practical embodiment, the distal end faces of the first and second return pieces 54, 74, including the first and second notches 60, 78, are approximately perpendicular to the plate surface. Accordingly, in the attached state shown in FIG. 2, the inner needle 26 and the first and second return pieces 54, 74 are approximately in line contact or point contact.

The curvature of the inner surfaces of the first and second notches 60, 78 is smaller than that of the outer circumferential surface of the inner needle 26, so that the contact range between the first and second return pieces 54, 74 and the inner needle 26 in the circumferential direction is made narrow, which also reduces resistance during the withdrawal. Thus, in this practical embodiment, the inner needle 26 and each of the first and second return pieces 54, 74 are approximately in point contact with each other at a single point, and the sliding resistance due to contact is sufficiently small.

The outer needle unit 14, which is separated from the inner needle unit 16, is placed as an indwelling needle in a state of puncture into a blood vessel, and functions as an access port to the blood vessel by an external circuit (not shown) being connected to the proximal end side of the outer needle hub 20, and is used, for example, as an administration port for transfusion solution or drug solution, a blood removal port or a blood return port for artificial dialysis, or the like.

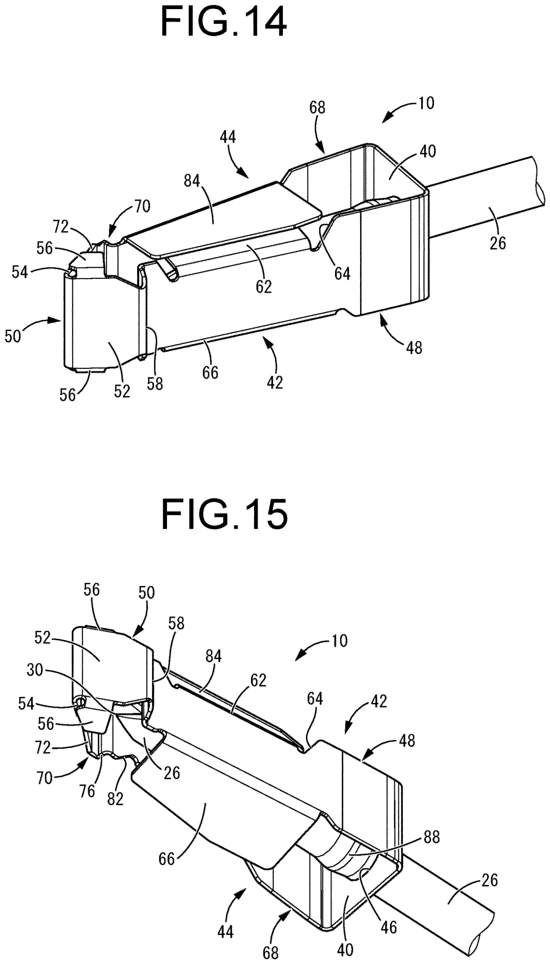

When the inner needle 26 is withdrawn from the outer needle 18, the needle tip 30 of the inner needle 26 is covered by the needle tip protector 10, which is moved to the needle tip 30 side with respect to the inner needle 26, as shown in FIGS. 14 to 17. That is, when the inner needle 26 moves toward the proximal end side with respect to the needle tip protector 10 whose movement to the proximal end side is limited by the outer needle hub 20, the contact between the outer circumferential surface of the inner needle 26 and the first and second return pieces 54, 74 is released, and the first and second side plates 42, 44 elastically recover their shape. By so doing, the first and second side plates 42, 44 incline so as to approach each other toward the distal end side, and the first needle-tip protection part 50 and the second needle-tip protection part 70 are displaced to a position where they both cover the distal end side of the inner needle 26, so that the needle-tip guarding part covering the needle tip 30 is constituted by the first and second needle-tip protection parts 50, 70. Accordingly, the needle tip 30 of the inner needle 26 is not exposed to the distal end side, and the movement of the inner needle 26 to the distal end side is limited by the contact with the first and second needle-tip protection parts 50, 70, thereby avoiding the risk of accidental puncture due to exposure of the inner needle 26.

In the needle-tip protection state by the needle tip protector 10 as described above, the first and second return pieces 54, 74 of the first and second needle-tip protection parts 50, 70 overlap each other on the distal end side of the needle tip 30. By doubly covering the distal end side of the inner needle 26 with the first and second needle-tip protection parts 50, 70, a high level of safety is ensured.

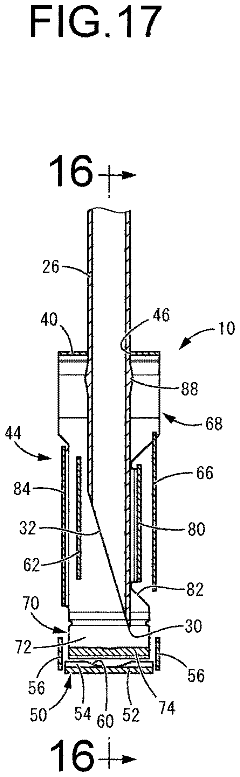

The needle tip 30 is covered by the pair of lateral guarding pieces 56, 56 provided to the first needle-tip protection part 50 so as to be less likely to be observed from the outside. Besides, even if the needle tip 30 moves to the distal end side, the pair of lateral guarding pieces 56, 56 prevents the needle tip 30 from coming off the first and second needle-tip protection parts 50,70 in the width direction (the vertical direction in FIG. 17) and being exposed to the outside.

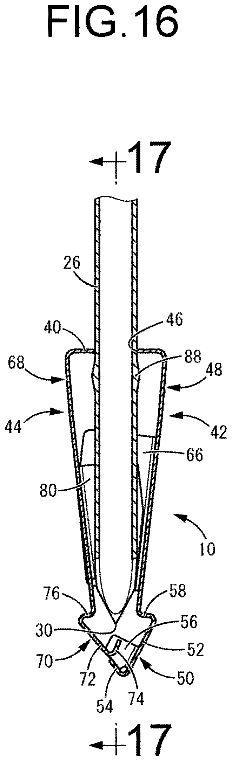

Furthermore, as shown in FIG. 16, the pair of lateral guarding pieces 56, 56 are located on the lateral side of the second distal-end protection piece 72 and the second return piece 74 in the needle-tip protection state. By so doing, the relative displacement of the first needle-tip protection part 50 and the second needle-tip protection part 70 in the lateral direction (the direction orthogonal to the paper surface in FIG. 16) is limited by the contact between the lateral guarding piece 56 and the second distal-end protection piece 72 as well as the second return piece 74. This prevents re-exposure of the needle tip 30 due to unintended relative displacement of the first and second needle-tip protection parts 50, 70 in the lateral direction.

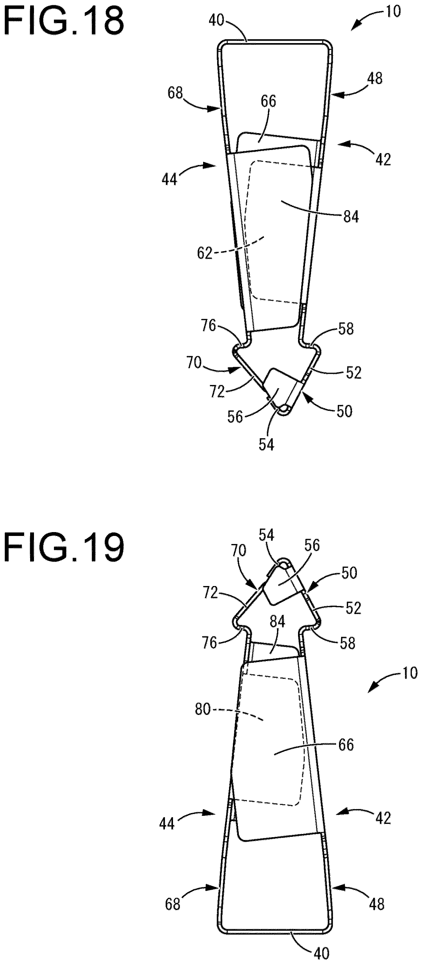

In the needle-tip protection state, as shown in FIGS. 18 and 19, the first and second main bodies 48, 68 of the first and second side plates 42, 44 approach each other toward the distal end, and the distal end portions of the first and second main bodies 48, 68 are close to the inner needle 26 in the left-right direction as shown in FIG. 16. This limits the amount of displacement (shake) of the inner needle 26 in the direction of facing of the first and second main bodies 48, 68 (the left-right direction), thereby preventing exposure of the needle tip 30 or the like due to displacement in the left-right direction.

In the needle-tip protection state, as shown in FIG. 17, the first and second lateral cover pieces 62, 80 provided to the first and second side plates 42, 44 are close to the distal end portion of the inner needle 26 in the vertical direction. Accordingly, the amount of displacement (shake) of the inner needle 26 is limited in the direction of facing of the first and second lateral cover pieces 62, 80 (the vertical direction), and exposure of the needle tip 30 or the like due to displacement in the vertical direction is prevented.

The first lateral cover piece 62 is provided to the first side plate 42, and the second lateral cover piece 80 is provided to the second side plate 44. This enables easy forming of the needle tip protector 10 by press working or the like, even if the distance between the facing surfaces of the first lateral cover piece 62 and the second lateral cover piece 80 is set small. Therefore, both the first and second lateral cover pieces 62, 80 can be positioned close enough to a thin inner needle 26 for medical use of, for example, about 16 to 24 G, thereby effectively limiting displacement of the inner needle 26 in the vertical direction. By setting the amount of shift of each center of the first side plate 42 and the second side plate 44 in the plate width direction according to the thickness (gauge number) of the inner needle 26, even when the needle tip protector 10 is used for a thin inner needle 26 of 24 G or the like, for example, both the first and second lateral cover pieces 62, 80 can be positioned close enough to the inner needle 26 while reliably obtaining the member strength of the needle tip protector 10.

In this practical embodiment, the first lateral cover piece 62 is provided protruding inward in the vertical direction at the first concave part 64 of the first side plate 42, and the second lateral cover piece 80 is provided protruding inward in the vertical direction at the second concave part 82 of the second side plate 44. With these arrangements, both the first and second lateral cover pieces 62, 80 can be positioned close to the inner needle 26.

The first side plate 42 is wide on both sides off the first concave part 64 in the front-back direction, thereby surely obtaining deformation rigidity. Besides, the first side plate 42 is integrally provided with the first lateral cover piece 62 protruding inward at the narrow portion where the first concave part 64 is formed, thereby affording deformation rigidity by the reinforcement effect of the first lateral cover piece 62. In the second side plate 44, similarly to the first side plate 42, deformation rigidity can be achieved at the portion where the second concave part 82 is formed and on both sides thereof in the front-back direction.

The first and second lateral cover pieces 62, 80 do not interfere with the first and second side plates 42, 44 in the needle-tip protection state where the first and second side plates 42, 44 incline, because the protruding distal ends from the first and second side plates 42, 44 have an inclined shape considering the needle-tip protection state. Therefore, the deformation (the shape recovery) of the first and second side plates 42, 44 reliably occurs without being hindered by the first and second lateral cover pieces 62, 80, and the needle tip protector 10 stably moves to the desired needle-tip protection mode.

The first side plate 42 protrudes to the outside of the second lateral cover piece 80 in the width direction, thereby sufficiently obtaining the width dimension and reliably obtaining strength such as deformation rigidity. In this way, if the first side plate 42 protrudes to the outside of the second lateral cover piece 80 in the width direction, there is a risk that the protection state of the needle tip 30 by the needle tip protector 10 may be released when the said protruding portion is pushed by hand or the like. Therefore, by providing the first protrusion piece 66 to the said protruding portion, it is difficult to push the said protruding portion, thereby stably maintaining the needle-tip protection state of the needle tip protector 10. In the needle-tip protection state, as shown in FIG. 19, the first protrusion piece 66 reaches a position where the first protrusion piece 66 covers the lateral side of the second side plate 44. Thus, even if the first protrusion piece 66 is pushed by a finger, the finger is caught by the second side plate 44 so that the first protrusion piece 66 is unlikely to be pushed in significantly, thereby making it difficult to release the needle-tip protection state.

Similarly, owing to the second protrusion piece 84 provided to the second side plate 44, in the needle-tip protection state, the portion of the second side plate 44 protruding to the outside of the first lateral cover piece 62 in the width direction is unlikely to be pushed, thereby avoiding the release of the needle-tip protection state. As shown in FIG. 18, the second protrusion piece 84 is provided at a protrusion height that slightly does not reach the first side plate 42, but because the protruding distal end is close to the first side plate 42, the second protrusion piece 84 is unlikely to be pushed in by a finger or the like catching on the first side plate 42, similarly to the first protrusion piece 66.

Meanwhile, the needle tip protector 10, which protects the needle tip 30 of the inner needle 26 in the state of being housed in the outer needle hub 20, is separated from the outer needle hub 20 and detached to the proximal end side as the inner needle 26 is withdrawn to the proximal end side. That is, the needle tip protector 10 in the needle-tip protection state deforms so that the first and second side plates 42, 44 approach each other toward the distal end. This releases the axial engagement of the first and second stepped parts 58, 76 with the locking shoulder part 24 of the outer needle hub 20 and allows the needle tip protector 10 to move to the proximal end side with respect to the outer needle hub 20.

As shown in FIGS. 12, 16, and 17, a large-diameter part 88 with a partially enlarged outer diameter dimension is provided near the distal end of the inner needle 26, and the outer diameter dimension of the large-diameter part 88 is larger than the inner diameter dimension of the insertion hole 46 formed in the bottom part 40 of the needle tip protector 10. With this configuration, when the inner needle 26 in the needle-tip protection state is pulled further toward the proximal end side, the large-diameter part 88 of the inner needle 26 is locked to the bottom part 40 of the needle tip protector 10 in the needle axis direction, and the needle tip protector 10 moves to the proximal end side together with the inner needle 26. Accordingly, with the needle tip 30 of the inner needle 26 protected, the needle tip protector 10 is separated from the outer needle hub 20, which is indwelled in a state of puncture into the blood vessel. In this way, with the inner needle unit 16 separated from the outer needle unit 14, the needle tip 30 of the inner needle 26 is covered by the needle tip protector 10. This makes it possible to prevent accidental touching of the needle tip 30 of the inner needle 26 when handling the inner needle unit 16 after separation, thereby ensuring safety.

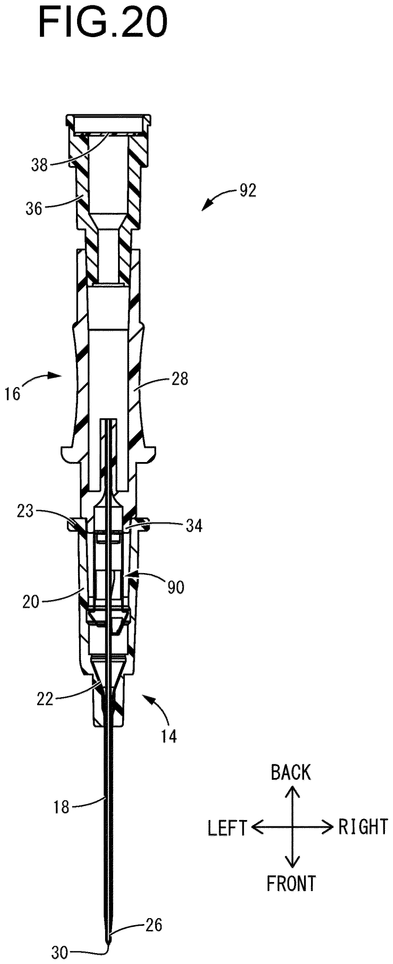

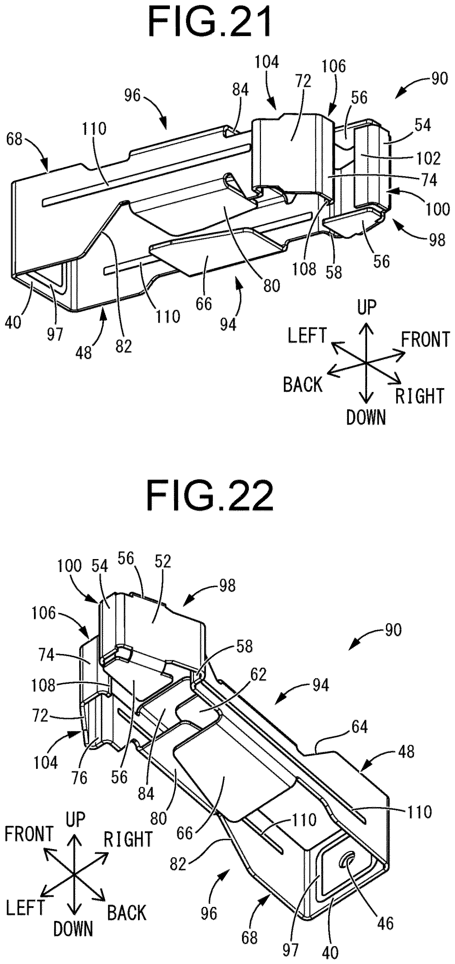

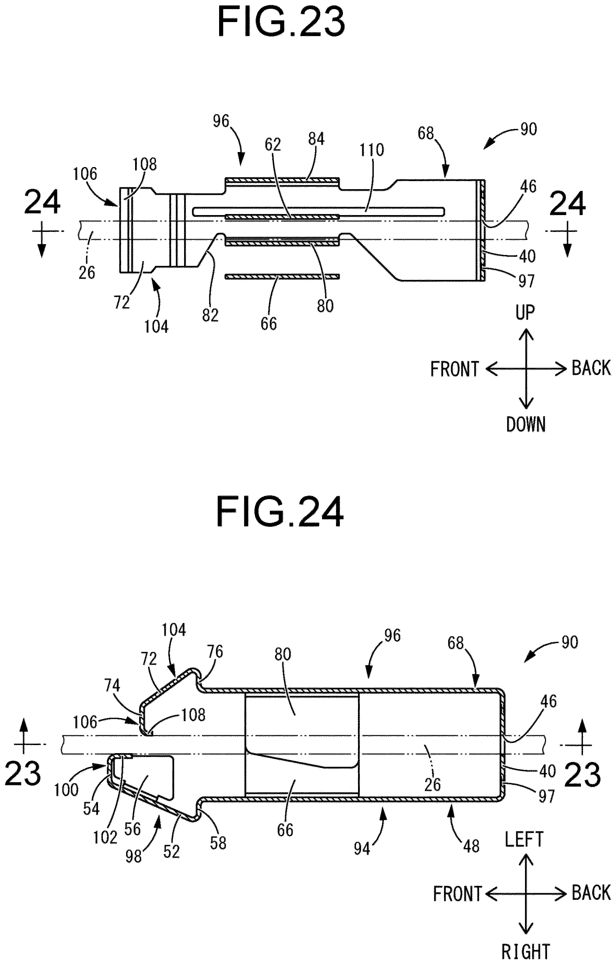

FIG. 20 shows a needle assembly 92 with a needle tip protector 90 according to a second practical embodiment of the present invention. The needle tip protector 90 is shown in FIGS. 21 to 24, and includes a first side plate 94 and a second side plate 96 extending from the bottom part 40 to the needle tip 30 side (the left side in FIG. 23). In the following description, components and parts that are substantially identical with those in the first practical embodiment will be assigned like symbols and not described in any detail.

The bottom part 40 of this practical embodiment is provided with a thin-walled part 97 serving as a stress transmission suppressing part and extending in an approximately quadrangular ring shape so as to surround the insertion hole 46. The bottom part 40 is recessed like a groove at the thin-walled part 97 on front and back faces. By providing the thin-walled part 97, for example, an external force acting from the radial outside of the thin-walled part 97 is less likely to be transferred to the radial inside. This prevents deformation of the radially inner portion of the bottom part 40 due to the action of the external force and the attendant deformation of the insertion hole 46 or the like. The thin-walled part 97 is formed, for example, by coining similar to a thin-walled part 110 described later. The diameter of the insertion hole 46 of the bottom part 40 is set according to the outer diameter of the inner needle 26, which is set small in this practical embodiment corresponding to the inner needle 26 thinner than that in the first practical embodiment.

The first side plate 94 has the same structure as the first side plate 42 of the first practical embodiment overall. The first side plate 94 has a structure in which a first needle-tip protection part 98 serving as a needle-tip guarding part is integrally formed on the distal end side of the first main body 48. The first needle-tip protection part 98 includes a first needle-tip cover part 100 extending from the protruding distal end of the first stepped part 58. The first needle-tip cover part 100 comprises the first distal-end protection piece 52 provided continuously with the first stepped part 58, and the first return piece 54 protruding from the distal end of the first distal-end protection piece 52. The angle formed between the first return piece 54 of this practical embodiment and the first distal-end protection piece 52 is larger than that in the first practical embodiment, and is an obtuse angle larger than 90°.

A first needle-tip stopper part 102 is provided at the protruding distal end of the first return piece 54. The first needle-tip stopper part 102 has a rectangular plate shape, and protrudes from the first return piece 54 toward the right side in FIG. 24, which is the needle base side. The first needle-tip stopper part 102 is formed by bending and is provided integrally and continuously with the first return piece 54.

The lateral guarding pieces 56, 56 extending from the first distal-end protection piece 52 are provided on the lateral side of the first needle-tip protection part 98. The lateral guarding piece 56 of this practical embodiment is provided with a length that reaches the first needle-tip stopper part 102, and has a larger width dimension in the left-right direction than that in the first practical embodiment. In this way, the lateral guarding piece 56 has a large width with a length that reaches the first needle-tip stopper part 102, thereby making it difficult for the needle tip 30 to be exposed to the lateral side.

The second side plate 96 has the same structure as the second side plate 44 of the first practical embodiment overall. The second side plate 96 has a structure in which a second needle-tip protection part 104 serving as a needle-tip guarding part is integrally formed on the distal end side of the second main body 68. The second needle-tip protection part 104 includes a second needle-tip cover part 106 extending from the protruding distal end of the second stepped part 76. The second needle-tip cover part 106 comprises the second distal-end protection piece 72 provided continuously with the second stepped part 76, and the second return piece 74 protruding from the distal end of the second distal-end protection piece 72. The angle formed between the second return piece 74 of this practical embodiment and the second distal-end protection piece 72 is larger than that in the preceding first practical embodiment, and is an obtuse angle larger than 90°.

A second needle-tip stopper part 108 is provided at the protruding distal end of the second return piece 74. The second needle-tip stopper part 108 has a rectangular plate shape, and protrudes from the second return piece 74 toward the right side in FIG. 24, which is the needle base side. The second needle-tip stopper part 108 is formed by bending and is provided integrally and continuously with the second return piece 74.

In FIG. 24, which shows the shape of the needle tip protector 90 attached to the inner needle 26, the protruding distal end of the first needle-tip stopper part 102 (the end on the needle base side) is located on the distal end side with respect to the second needle-tip protection part 104. Besides, the length dimension of the first needle-tip stopper part 102 is longer than that of the second needle-tip stopper part 108. It is desirable that the axial distance between the first needle-tip stopper part 102 and the second needle-tip protection part 104 be smaller than the length dimension of the first needle-tip stopper part 102.

In this practical embodiment, the first main body 48 of the first side plate 94 and the second main body 68 of the second side plate 96 each include a thin-walled part 110 extending linearly in the front-back direction. The thin-walled parts 110 are formed by coining (light forging by pressing of a die). The thin-walled parts 110 improve deformation rigidity of the first and second main bodies 48, 68, thereby preventing unintended opening of the first and second side plates 94, 96 in the protection state of the needle tip 30 described later.

As shown in FIG. 20, the needle tip protector 90 is attached to the inner needle 26. The inner needle 26 is inserted between the first side plate 94 and the second side plate 96, and the first and second return pieces 54, 74 and the first and second needle-tip stopper parts 102, 108 are in contact with the outer circumferential surface of the inner needle 26. With this configuration, the first side plate 94 and the second side plate 96 are displaced to the sides such that they are separated from each other in the left-right direction, and an urging force in the direction of mutual approach is exerted on the first side plate 94 and the second side plate 96. As shown in FIG. 23, in comparison with the first practical embodiment, the first and second lateral cover pieces 62, 80 of this practical embodiment are arranged with a shorter distance therebetween and in positions closer to the inner needle 26, so that the allowable amount of movement of the inner needle 26 in the vertical direction is smaller.

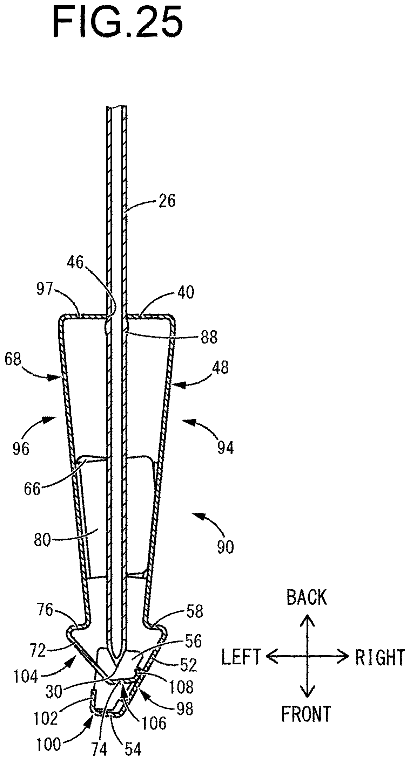

When the inner needle 26 is withdrawn from the outer needle 18, as shown in FIG. 25, the needle tip 30 of the inner needle 26 is covered and protected by the needle tip protector 90 that has moved to the needle tip 30 side. That is, the first and second return pieces 54, 74 and the first and second needle-tip stopper parts 102, 108 are released from contact with the inner needle 26, which displaces the first and second needle-tip protection parts 98, 104 so as to approach each other, so that the first and second needle-tip cover parts 100, 106 move to the extension of the inner needle 26 on the needle tip 30 side. By so doing, the distal end side of the needle tip 30 is covered by the first and second needle-tip cover parts 100, 106, and the needle tip 30 of the inner needle 26 is in the needle-tip protection state by the needle tip protector 90. Since the first needle-tip stopper part 102 is located at the distal end side without being caught by the second needle-tip cover part 106, the first needle-tip stopper part 102 does not interfere with the approaching displacement of the first and second side plates 94, 96.

As shown in FIG. 25, with the needle tip 30 protected by the needle tip protector 90, the protruding distal end of the second lateral cover piece 80 is pressed against the first main body 48 of the first side plate 94. Similarly, the protruding distal end of the first lateral cover piece 62 is pressed against the second main body 68 of the second side plate 96. With this arrangement, the urging force in the direction of mutual approach of the first side plate 94 and the second side plate 96 is set greater, and displacement of the first side plate 94 and the second side plate 96 in the direction of mutual separation by the action of an external force is unlikely to occur.

Besides, with the needle tip 30 protected by the needle tip protector 90, the connected portion between the second return piece 74 and the second needle-tip stopper part 108 is pressed against the first distal-end protection piece 52. This defines the amount of relative approaching displacement of the first and second needle-tip protection parts 98, 104. For example, by reliably obtaining a gap between the inner needle 26 and the first and second stepped parts 58, 76, the inner needle 26 is unlikely to push open the first and second needle-tip protection parts 98, 104.

With the needle tip 30 protected as shown in FIG. 25, the lateral guarding pieces 56, 56 extend out to the proximal end side beyond the second needle-tip cover part 106. With this configuration, the needle tip 30 covered by the second needle-tip cover part 106 is located between the facing surfaces of the lateral guarding pieces 56, 56, and is prevented from being exposed to the outside. In particular, the lateral guarding pieces 56, 56 are provided so as to cover as far as the connected portion between the second return piece 74 and the second needle-tip stopper part 108. Thus, even if the needle tip 30 moves along the second return piece 74 until it comes into contact with the second needle-tip stopper part 108, the needle tip 30 is kept covered by the lateral guarding pieces 56, 56 without being exposed to the lateral side.

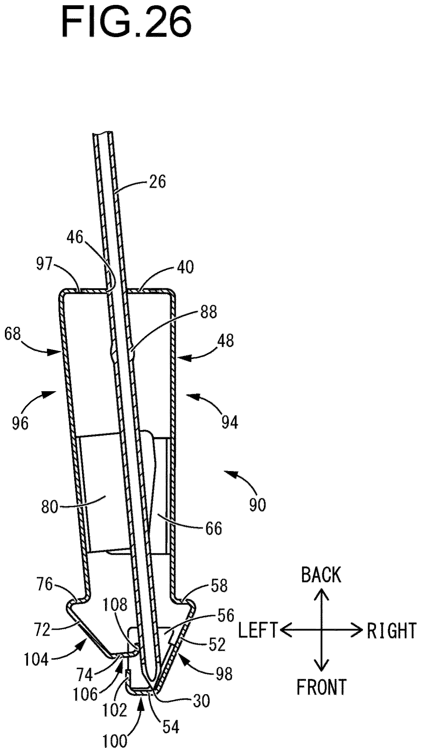

Meanwhile, with the needle tip 30 protected by the needle tip protector 90, it is conceivable that due to the inner needle 26 moving relative to the needle tip protector 90, the needle tip 30 may come off the second needle-tip cover part 106 and be abutted against the first needle-tip cover part 100, as shown in FIG. 26. In this state, if the needle tip 30 moves along the first return piece 54 to the second side plate 96 side (upward in FIG. 26), there is a risk that the needle tip 30 may come off to the protruding distal end side of the first return piece 54 while pushing the second side plate 96 away. Therefore, in this practical embodiment, the first needle-tip stopper part 102 is provided at the protruding distal end of the first return piece 54. By the needle tip 30 that moves along the first return piece 54 being locked by the first needle-tip stopper part 102, the needle tip 30 is prevented from coming off to the protruding distal end side of the first return piece 54. With this configuration, the needle tip 30 is kept in a protection state covered by the first needle-tip cover part 100 to prevent accidental puncture or the like.

In this practical embodiment, the second needle-tip stopper part 108 is provided at the protruding distal end of the second return piece 74, making it difficult for the needle tip 30 to come off the second needle-tip cover part 106 to the protruding distal end side of the second return piece 74. Hence, even if the needle tip 30 of the inner needle 26 moves in the left-right direction with respect to the needle tip protector 90, the protection state of the needle tip 30 is maintained.

The protruding dimension of the second needle-tip stopper part 108 is smaller than the protruding dimension of the first needle-tip stopper part 102. With this configuration, with the needle tip 30 protected as shown in FIG. 25, the needle tip 30 is unlikely to enter between the second needle-tip stopper part 108 and the first distal-end protection piece 52. Even if the needle tip 30 enters between the second needle-tip stopper part 108 and the first distal-end protection piece 52, the transition to the state shown in FIG. 26 due to the inner needle 26 pressing the second needle-tip stopper part 108 is unlikely to occur.

As can be seen by comparing FIG. 24 of this practical embodiment with FIG. 2 of the first practical embodiment, the contact angle of the needle-tip guarding parts 98, 104 (50, 70) at the contact portion with the inner needle 26 in the state of being externally mounted about the inner needle 26 is different from that in the first practical embodiment. In the first practical embodiment, the contact portion with the inner needle 26 is the distal end face of the return pieces 54, 74, which approach the inner needle 26 at an angle from the diagonal direction. On the other hand, in this practical embodiment, the needle-tip stopper parts 102, 108 provided at the distal ends of the needle-tip guarding parts 98, 104 extend approximately parallel to the inner needle 26, and each surface of the needle-tip stopper parts 102, 108 comprises a contact portion with the inner needle 26. Then, as can be seen by comparing FIG. 25 of this practical embodiment with FIG. 16 of the first practical embodiment, with the needle tip 30 protected by the needle tip protector 90 (10), in the first practical embodiment, the first return pieces 54, 74 located at the most distal end of the needle-tip guarding parts 50, 70 incline to the needle hub side, but are heading in the direction in which the needle tip moves to be exposed (leftward in FIG. 16 for the needle-tip guarding part 50, and rightward in FIG. 16 for the needle-tip guarding part 70), thereby failing to form effective return portions. On the other hand, in this practical embodiment, the needle-tip stopper parts 102, 108 located at the most distal end of the needle-tip guarding parts 98, 104 incline in the opposite direction to the direction in which the needle tip moves to be exposed (leftward in FIG. 25 for the needle-tip guarding part 98, and rightward in FIG. 25 for the needle-tip guarding part 104), thereby forming effective return portions.

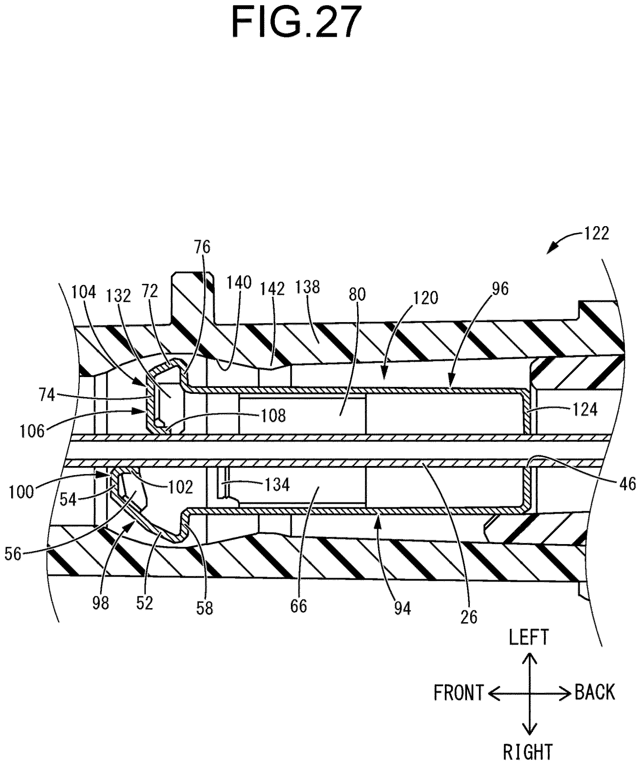

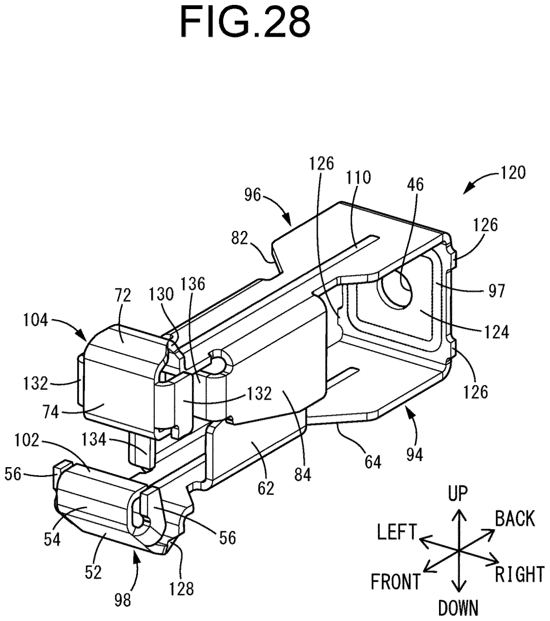

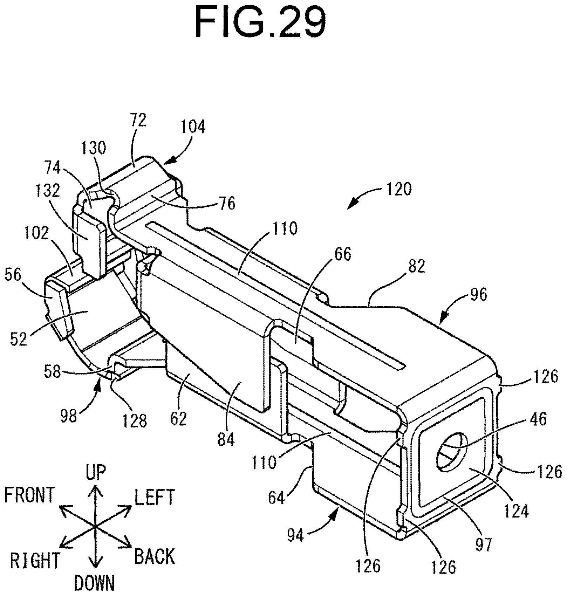

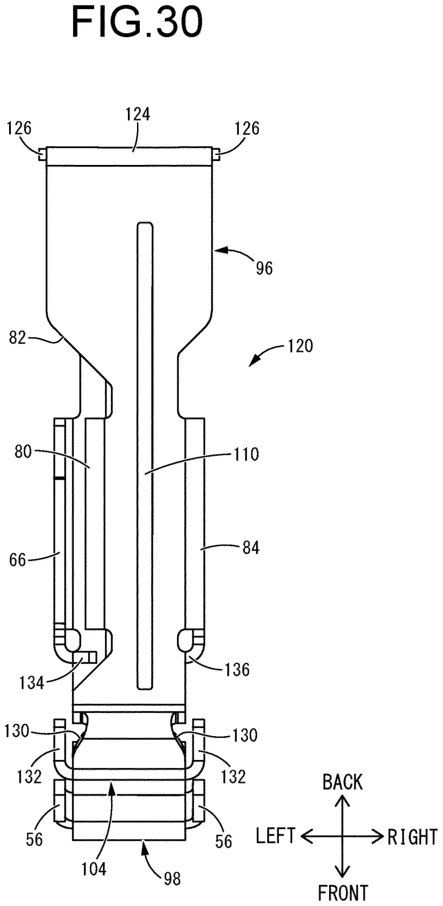

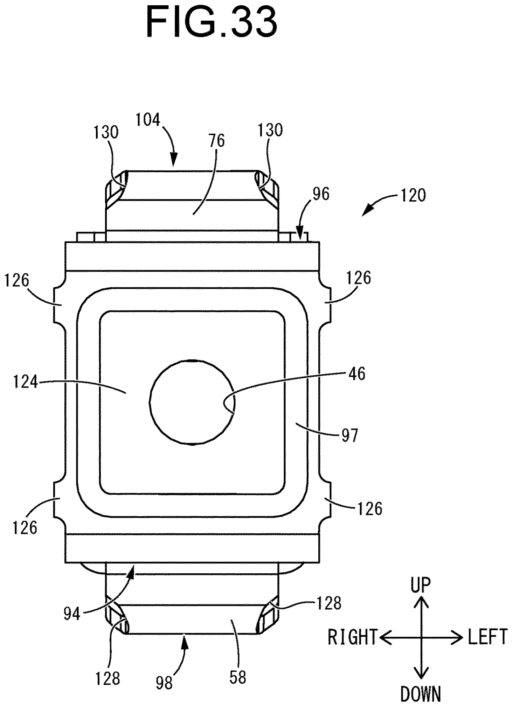

FIG. 27 shows a portion of a needle assembly 122 including a needle tip protector 120 according to a third practical embodiment of the present invention. As also shown in FIGS. 28 to 33, the needle tip protector 120 includes a bottom part 124 located on the needle base side of the inner needle 26 (the left side in FIG. 30), and a first side plate 94 and a second side plate 96 extending out from the bottom part 124 to the needle tip 30 side (the right side in FIG. 30). The needle tip protector 120 of this practical embodiment is, when housed in an outer needle hub 138 (described below) shown in FIG. 27, housed in an orientation rotated by 90° around the central axis (the needle axis) compared to the first and second practical embodiments. Therefore, in the following description, the vertical direction of the needle tip protector 120 corresponds to the left-right direction of the needle tip protector 90, and the left-right direction of the needle tip protector 120 corresponds to the vertical direction of the needle tip protector 90 of the second practical embodiment.

The bottom part 124 has an approximately rectangular flat-plate shape overall similar to the bottom part 40 of the second practical embodiment, with the first side plate 94 and the second side plate 96 integrally formed so as to rise from the upper and lower facing edges to the needle tip 30 side (the front side). The bottom part 124 is penetrated by an insertion hole 46 at the center portion in the plate thickness direction. The bottom part 124 includes convex parts 126 protruding leftward or rightward from the facing edges on the left and right sides. Two convex parts 126 are formed at mutually separated positions in the vertical direction on each of the left and right facing edges of the bottom part 124, and a total of four convex parts 126 are formed on the left and right sides. It would be acceptable as long as the convex parts 126 are provided separately on the left and right facing edges, and the number and arrangement of the convex parts 126 on each of the left and right facing edges are not particularly limited.

The needle tip protector 120 is a press-formed product. When the needle tip protector 120 is manufactured by press working of a blank metal plate, it is first separated from the blank metal plate at portions other than the four convex parts 126, 126, 126, 126. This separation (press cutting) from the blank metal plate at the positions away from the convex parts 126 enables highly precise cutting without burrs or distortion, because the cutting is performed on a blank metal plate of flat plate shape before stresses such as bending are applied thereto. Next, each part of the needle tip protector 120 is formed by bending process while maintaining continuity with the blank metal plate at each convex part 126. Each convex part 126 is then cut to produce the needle tip protector 120. Since the bending process is performed prior to the cutting of the convex part 126, the cut portion of the convex part 126 is more likely to generate burrs during cutting than other cut portions that are cut before the bending process, and the shape and dimensional accuracy are likely to be lower. That is, in this practical embodiment, the portions where burrs are generated or processing accuracy is low when separating from the blank metal plate are limited to the convex parts 126, and burrs are prevented and processing accuracy is improved in the cut portions other than the convex part 126. Since the portions with low processing accuracy is limited to the convex part 126, post-processing such as chamfering and polishing can be performed on the convex part 126 as necessary to achieve high accuracy in dimension and shape throughout the entire cutting portion while reducing the labor required for post-processing.

In manufacturing the needle tip protector 120, it is desirable that, with portions other than each convex part 126 separated from the blank metal plate, the first and second side plates 94, 96, etc. be formed by press bending process while the bottom part 124 be curved into a bow shape that is convex forward toward the vertical center. By so doing, by separating each convex part 126 from the blank metal plate and releasing the curved deformation of the bottom part 124, the inclination angle of the first and second side plates 94, 96 with respect to the bottom part 124 can be set to be smaller than 90° without increasing the bending angle of the first and second side plates 94, 96 with respect to the blank metal plate. Therefore, the shape of the needle tip protector 120 in the needle-tip protection state described later can be easily realized. In this practical embodiment, the convex parts 126 are formed on both end portions in the left-right direction, which makes it easier to bend the bottom part 124 into a bow shape between the convex parts 126, 126 on the left and right sides. Even if the bottom part 124 is slightly curved as a result of the press bending process while being curved into a bow shape as described above, the bottom part 124 can be regarded as a substantially flat-plate shape. The bottom part 124 may also be made into a plate shape that is actively curved by bending process, etc.

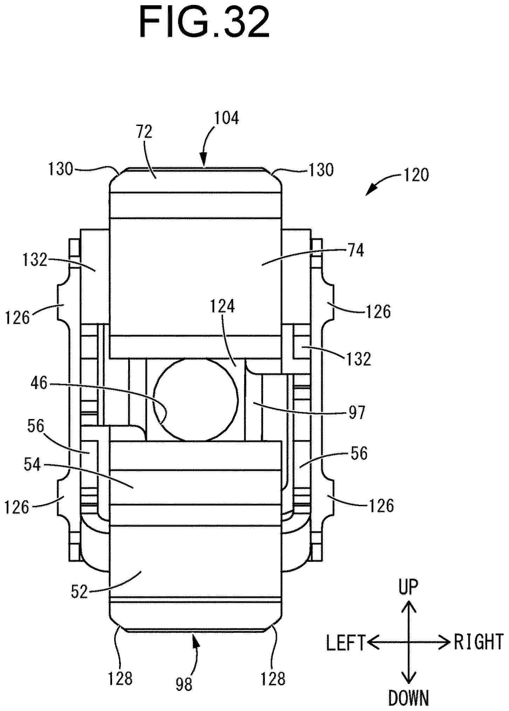

In the continuous portion between the first distal-end protection piece 52 and the first stepped part 58, which is the proximal end of the first needle-tip protection part 98, as shown in FIGS. 28 to 30, first relief parts 128 serving as relief parts are formed at respective ends of the first side plate 94 in the left-right direction, which is the plate width direction thereof. The first relief part 128 has a shape of a notch (in this practical embodiment, a curved, concave notch) that opens outward in the left-right direction, and has an expanded shape with a front-back width dimension increasing outward in the left-right direction. With such first relief parts 128 formed, as shown in FIG. 32, the left and right both ends of the continuous portion between the first distal-end protection piece 52 and the first stepped part 58, which is the outermost corners of the first needle-tip protection part 98, have an outline of a curve in the projected view in the needle-axis direction, in comparison with a case where the first relief parts 128 do not exist, as shown hypothetically in the chain double-dashed line. That is, the continuous portion between the first distal-end protection piece 52 and the first stepped part 58 in the first needle-tip protection part 98 is necked and made narrow in the left-right direction. With this configuration, even if the continuous portion between the first distal-end protection piece 52 and the first stepped part 58 comes into contact (interferes) with the inner circumferential surface of the outer needle hub 138, the interfering portion on the inner circumferential surface of the outer needle hub 138 is unlikely to be scraped by the needle tip protector 120. Even if the first needle-tip protection part 98 comes into contact with the inner circumferential surface of the outer needle hub 138 at the portion forming the first relief part 128, the contact portion is chamfered when viewed in the needle axis direction by the formation of the curved first relief part 128. Thus, a wide contact surface can be ensured, whereby damages such as scratches to the inner circumferential surface of the outer needle hub 138 are unlikely to be caused.

Similarly, in the continuous portion between the second distal-end protection piece 72 and the second stepped part 76, which is the proximal end of the second needle-tip protection part 104, second relief parts 130 serving as relief parts are formed at respective ends in the left-right direction. The formation of the second relief part 130, which has a shape of a notch, makes the proximal end portion of the second needle-tip protection part 104 narrow in a necked shape. This produces the same effect in the continuous portion between the second distal-end protection piece 72 and the second stepped part 76 including the second relief parts 130 as in the continuous portion between the first distal-end protection piece 52 and the first stepped part 58 including the first relief parts 128. The first relief part 128 and the second relief part 130 may have approximately the same shape and size as each other, or they may have different shapes and sizes from each other. For example, the second relief part 130 provided on the short second side plate 96 side may be smaller than the first relief part 128 provided on the long first side plate 94 side.

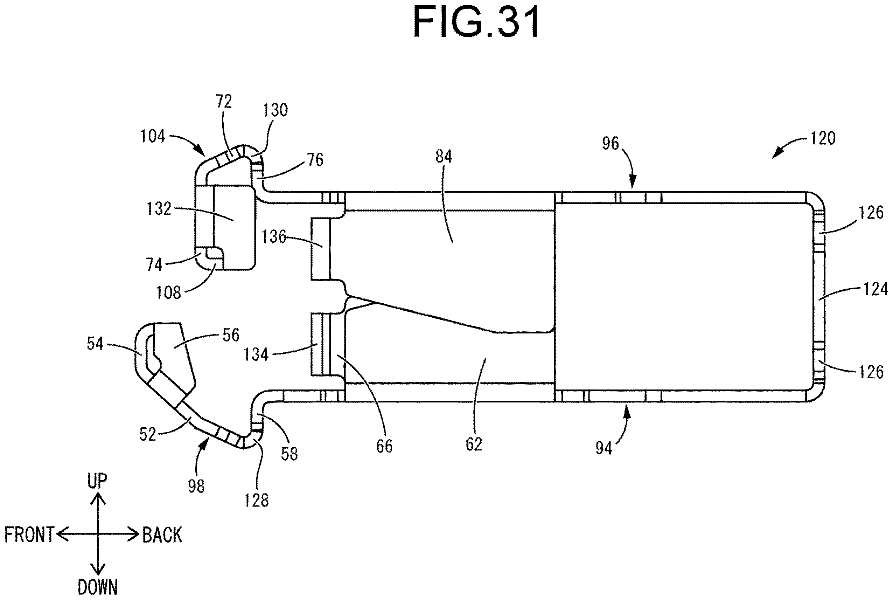

In this practical embodiment of the needle tip protector 120, the second needle-tip protection part 104 of the second side plate 96 includes lateral guarding pieces 132 covering the left and right lateral sides of the second needle-tip protection part 104, similarly to the lateral guarding piece 56 of the first side plate 94. As shown in FIGS. 28 and 31, the lateral guarding piece 132 extends from the second return piece 74 of the second needle-tip protection part 104. In the second needle-tip protection part 104 of this practical embodiment, the second distal-end protection piece 72 has a shorter length and the second return piece 74 has a longer length, compared to the second practical embodiment.

The first protrusion piece 66 serving as a lateral regulating piece that extends from the first side plate 94 between the facing surfaces of the first side plate 94 and the second side plate 96 includes a first regulating protrusion 134 serving as a regulating protrusion. As shown in FIGS. 28 to 31, the first regulating protrusion 134 protrudes inward in the left-right direction toward the inner needle 26 on the front side of the first protrusion piece 66. The first regulating protrusion 134 of this practical embodiment is formed integrally at the front end of the first protrusion piece 66 by the front end portion of the first protrusion piece 66 being bent inward in the left-right direction to extend out. In the first regulating protrusion 134, the protruding distal end on the inside in the left-right direction is located close to the outer circumferential surface of the inner needle 26 while being spaced apart in the left-right direction.

The second protrusion piece 84 serving as a lateral regulating piece that extends from the second side plate 96 between the facing surfaces of the first side plate 94 and the second side plate 96 includes a second regulating protrusion 136 serving as a regulating protrusion protruding toward the inner needle 26 side. The second regulating protrusion 136 differs from the first regulating protrusion 134 in that it is integrally formed at the front end of the second protrusion piece 84, and protrudes opposite in the left-right direction. In the state before needle-tip protection operation of the needle tip protector 120 as shown in FIGS. 28 to 33, the first regulating protrusion 134 and the second regulating protrusion 136 are located apart from each other in the left-right direction. It is desirable that the first and second regulating protrusions 134, 136 be formed integrally by bending process as in this practical embodiment. However, they may be provided, for example, by partially thickening the distal end portions of the first and second protrusion pieces 66, 84 by means of padding or the like, or may be attached to the distal end portions of the first and second protrusion pieces 66, 84 as separate components. Considering the position regulating effect by contact with the inner needle 26, only one of the first and second regulating protrusions (134, 136) may be provided, and they need not be provided at the distal end of each protrusion piece. Furthermore, the specific formation position, shape and the like of the lateral regulating piece, which extends out between the facing surfaces of the first and second side plates 94, 96 and comprises the said protrusion piece, are not limited.

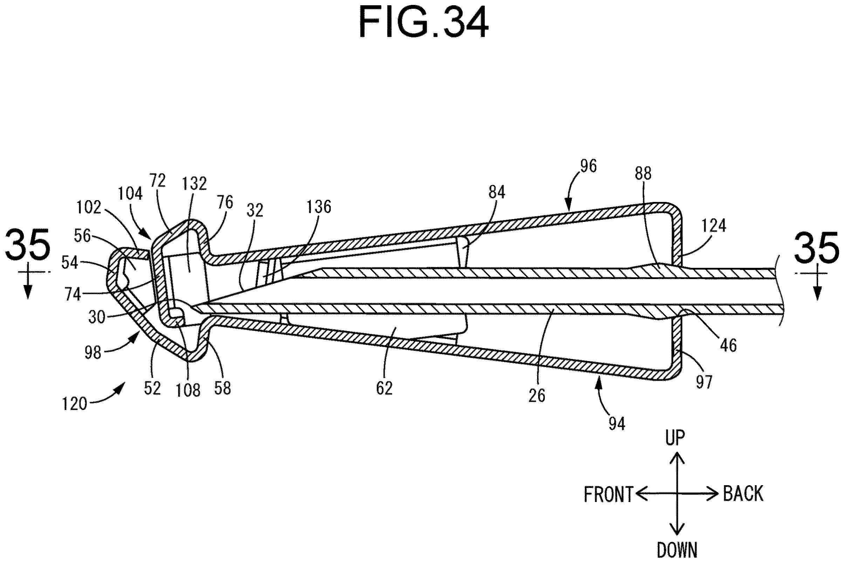

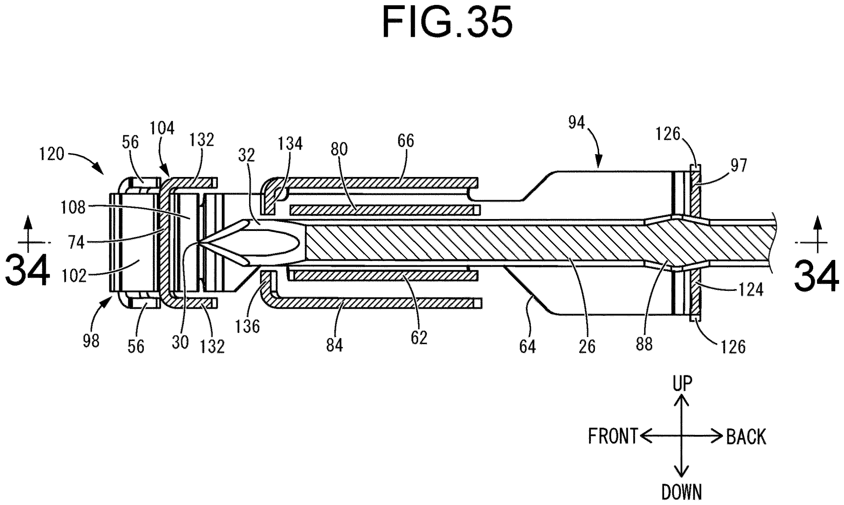

As shown in FIG. 27, the needle tip protector 120 is housed in the radial inside of the outer needle hub 138, and is externally mounted about the inner needle 26. Like the second practical embodiment, the needle tip protector 120 is configured such that by the inner needle 26 being inserted between the first and second needle-tip protection parts 98, 104, the first and second side plates 94, 96 are separated from each other in the direction of facing, and the first and second needle-tip protection parts 98, 104 are pressed against the inner needle 26 by the elasticity of the first and second side plates 94, 96 and the bottom part 124. As shown in FIGS. 34 and 35 described later, in this practical embodiment, the circumferential orientation of the needle tip protector 120 with respect to the blade surface 32 of the inner needle 26 is made different by being rotated by 90° with respect to those in the preceding first and second practical embodiments, with the blade surface 32 facing the second side plate 96 side. The circumferential orientation of the needle tip protector 120 with respect to the inner needle 26 is not particularly limited.

The outer needle hub 138 of this practical embodiment includes an expanded housing part 140 which is partially enlarged in diameter on the radial inside, and the first and second needle-tip protection parts 98, 104, which are mutually separated by being externally mounted about the inner needle 26, are housed in the expanded housing part 140. The maximum inner diameter dimension of the outer needle hub 138 at the formation zone of the expanded housing part 140 is larger than the maximum outer diameter dimension of the first and second needle-tip protection parts 98, 104 of the needle tip protector 120 mounted onto the inner needle 26. The expanded housing part 140 has a maximum inner diameter dimension in the middle in the axial direction. The inner circumferential surface of the expanded housing part 140 comprises a tapered surface that slopes radially inward as it goes away from the maximum inner diameter portion in the axial direction, and in this practical embodiment, has a concave curved surface that opens radially inward. In particular, the inner circumferential surface of the expanded housing part 140 has a sufficiently small inclination angle of the tapered surface on the axially proximal end side with respect to the maximum inner diameter portion, which is smaller than that of the tapered surface on the axially distal end side with respect to the maximum inner diameter portion. This makes it easy to remove the mold for forming the inner circumferential surface of the outer needle hub 138 from the outer needle hub 138 by climbing over a locking protrusion 142, which is described later, to the proximal end side.

A locking protrusion 142 protruding from the inner circumferential surface is formed at the proximal end side (the rear side) of the expanded housing part 140 in the outer needle hub 138. The locking protrusion 142 may have an annular shape that is provided continuously about the entire circumference, or may be provided partially in the circumferential direction, and may be provided in plurality in the circumferential direction. The locking protrusion 142 may have a cross-sectional shape that is approximately constant in the circumferential direction, or the cross-sectional shape may vary in the circumferential direction. The minimum inner diameter dimension of the outer needle hub 138 at the formation zone of the locking protrusion 142 is smaller than the diameter dimension through which the first and second needle-tip protection parts 98, 104 of the needle tip protector 120 attached to the inner needle 26 can pass. With this configuration, when the inner needle 26 is withdrawn to the proximal end side, the needle tip protector 120, which is locked to the locking protrusion 142 at the first and second needle-tip protection parts 98, 104, will move to the needle tip 30 side relative to the inner needle 26. Besides, the needle tip protector 120 in the needle-tip protection state described later can pass through the radial inside of the locking protrusion 142 because the first and second needle-tip protection parts 98, 104 are close to each other. When inserting the needle tip protector 120 into the radial inside of the outer needle hub 138, by positioning the second needle-tip stopper part 108 on the blade surface 32 and inserting the needle tip protector 120 with the first and second needle-tip protection parts 98, 104 being close to each other in the vertical direction, it is possible to insert the first and second needle-tip protection parts 98, 104 of the needle tip protector 120 attached to the inner needle 26 into the distal end side beyond the locking protrusion 142.

The first and second needle-tip protection parts 98, 104 of the needle tip protector 120 are easy to be inserted into the outer needle hub 138 because the maximum outer diameter dimension is reduced by the formation of the first and second relief parts 128, 130. Besides, even if the first and second needle-tip protection parts 98, 104 comes into contact with the inner circumferential surface of the outer needle hub 138 at the portions forming the first and second relief parts 128, 130 when the needle tip protector 120 is inserted into the outer needle hub 138, the portions of the first and second needle-tip protection parts 98, 104 having the maximum outer diameter are chamfered by the formation of the first and second relief parts 128, 130. This reduces the contact pressure and prevents damage to the inner circumferential surface of the outer needle hub 138.

The bottom part 124 of the needle tip protector 120 protrudes outward most in the left-right direction at the portions where the convex parts 126 are formed, so that the convex parts 126 are likely to come into contact with the radial inside of the outer needle hub 138. Therefore, it is possible to prevent damage to the outer needle hub 138 due to contact of the bottom part 124 by post-processing the convex parts 126 with a chamfer or the like, which is easier than post-processing the entire left and right edges of the bottom part 124.

When the inner needle 26 is withdrawn to the proximal end side with respect to the outer needle hub 138, as shown in FIGS. 34 and 35, the needle tip protector 120, which is moved to the needle tip 30 side of the inner needle 26, houses and protects the needle tip 30 of the inner needle 26. By so doing, the needle tip 30 of the inner needle 26 after use will not be exposed so that accidental contact with the needle tip 30 is prevented, thereby ensuring safety. With regard to the operation and protection mode of the needle tip protector 120 when protecting the needle tip 30, the explanation concerning approximately the same points as in the second practical embodiment is omitted.

The needle tip protector 120 of this practical embodiment not only includes the lateral guarding piece 56 in the first needle-tip protection part 98, but also includes the lateral guarding piece 132 in the second needle-tip protection part 104. Therefore, even if the lateral guarding piece 56 of the first needle-tip protection part 98 moves away from the upper and lower lateral side of the second needle-tip protection part 104, the needle tip 30 is unlikely to be exposed from the second needle-tip protection part 104 to the left and right lateral side, thereby achieving more reliable protection of the needle tip 30.

With the needle tip 30 protected by the needle tip protector 120, the first and second side plates 94, 96 approach each other. Thus, as shown in FIG. 35, the first and second regulating protrusions 134, 136 provided to the first and second protrusion pieces 66, 84 are located close to the inner needle 26 on the left and right sides thereof, with a prescribed distance. With this arrangement, even if the inner needle 26 moves to a position vertically outward from the first and second lateral cover pieces 62, 80, the tilting of the inner needle 26 in the right and left directions will be quickly regulated by contact with the first and second regulating protrusions 134, 136 in the initial stage of tilting where the inclination angle is still small. Therefore, exposure of the needle tip 30 to the left and right lateral side is more reliably prevented.

While the present invention has been described in detail hereinabove in terms of the practical embodiments, the invention is not limited by the specific description thereof. For example, the first and second protrusion pieces 66, 84 are not essential, and only one of them may be provided, or both of them may be omitted. Besides, it is desirable that the length dimension of the first and second protrusion pieces 66, 84 be large in order to effectively prevent, for example, the widthwise end of the first and second side plates 42, 44 from being pushed to forcibly release the needle-tip protection state. However, the length dimension of the first and second protrusion pieces 66, 84 can be smaller than those of the first and second lateral cover pieces 62, 80.

The specific structure of the first and second needle-tip protection parts 50, 70 is not particularly limited. That is, the first needle-tip protection part 50 of the preceding practical embodiment exemplifies a structure having the first distal-end protection piece 52 and the first return piece 54, each of which has an inclined shape. However, it would also be possible to provide, for example, a plate-shaped first needle-tip protection part protruding toward the inner needle 26 at the distal end of the first main body 48, approximately orthogonal to the needle axis direction. In this case, when the needle tip protector is attached to the inner needle 26, the protruding distal end of the first needle-tip protection part is in contact with the outer circumferential surface of the inner needle 26, and when the inner needle 26 is withdrawn, the first needle-tip protection part covers the distal end side of the inner needle 26 thereby protecting the needle tip 30. The same structure can be adopted for the second needle-tip protection part.

It is desirable that the center of first side plate 42 in the plate width direction at the formation zone of the first lateral cover piece 62 and the first protrusion piece 66, and the center of the second side plate 44 in the plate width direction at the formation zone of the second lateral cover piece 80 and the second protrusion piece 84 be mutually biased to the opposite side of the corresponding lateral cover piece 62, 80. However, the said center of the first side plate 42 and the said center of the second side plate 44 may be set to the locations that approximately coincide with each other in the width direction.

In the preceding first practical embodiment, the first and second lateral cover pieces 62, 80 are provided within the notch-shaped first and second concave parts 64, 82 opening at the side ends of the first and second side plates 42, 44 in the plate width direction. However, the first and second concave parts 64, 82 are not essential. In the preceding sixth preferred embodiment, at least one of the first and second concave parts 64, 82 is provided, and at least one of the first and second lateral cover pieces 62, 80 protrudes from the corresponding one of the first and second concave parts 64, 82.

The inner surface shape of the first and second notches 60, 78 is suitably a curved surface with the widthwise center portion being the deepest part, as shown in the preceding first practical embodiment. However, the said inner surface may, for example, extend in the width direction with an approximately constant depth dimension, or have a linear shape with the depth dimension increasing from widthwise both sides to the widthwise center with an approximately constant rate of change.

In the preceding first practical embodiment, an example is shown in which the notches (the first and second notches 60, 78) are formed in both the first and second side plates 42, 44. However, the notch may be provided in only one of the first and second side plates 42, 44, or in some cases, the notch is not provided in either of the first and second side plates 42, 44.

The preceding first practical embodiment illustrates that, when the inner needle 26 is withdrawn from the outer needle 18, the first and second side plates 42, 44 (the first and second return pieces 54, 74) of the needle tip protector 10 are released from contact with the inner needle 26, so that the first and second side plates 42, 44 move to the needle-tip protection state due to elastic recovery force (spring force). However, the transition of the needle tip protector 10 to the needle-tip protection state is not necessarily limited to deformation based on the elastic recovery force of the needle tip protector 10. Specifically, for example, a ring member may be arranged in the inside of the outer needle hub 20 on the proximal end side with respect to the needle tip protector 10. By the needle tip protector 10 moving to the proximal end side due to its engagement with the large-diameter part 88 of the inner needle 26 and being inserted into the ring member, the first and second side plates 42, 44 are brought closer to each other by the ring member, whereby the needle tip protector moves to the needle-tip protection state. In this case, the ring member is detachable from the outer needle hub 20 in the state of being externally placed around the needle tip protector 10 to hold the needle tip protector 10 in the needle-tip protection state.

In the preceding fifth or sixth preferred embodiment, the lateral cover piece may be provided on only one side of the puncture needle in the plate width direction. In the practical embodiments according to the preceding fifth or sixth preferred embodiment, for example, a pair of lateral cover pieces may be provided at both edges in the plate width direction of either one of the first side plate and the second side plate.

The preceding second practical embodiment illustrates an example in which both the first needle-tip cover part 100 and the second needle-tip cover part 106 include the respective needle-tip stopper parts. However, the needle-tip stopper part may be provided on only one of the first needle-tip cover part 100 and the second needle-tip cover part 106.

The present invention includes a thirteenth preferred embodiment in which the needle tip protector is attached to a puncture needle and prevents exposure of a needle tip by moving to the needle tip side, the needle tip protector including a first side plate part and a second side plate part extending from a bottom part located on the needle base side of the puncture needle to the needle tip side of the puncture needle. Distal end portions of the first side plate part and the second side plate part approach relative to each other so as to constitute a needle-tip guarding part that covers the needle tip. A distal end edge of at least one of the first side plate and the second side plate includes a notch extending in the plate width direction, and with the needle tip protector attached to the puncture needle, the puncture needle is configured to enter the notch to be positioned therein.

That is, the needle tip protector described in the above-mentioned Patent Document 1 has a structure in which a concave groove extending in the needle axis direction is formed in order to suppress lateral positioning deviation with respect to the puncture needle in the mounted state onto the puncture needle and during withdrawal of the puncture needle, and the puncture needle enters the bottom part of the concave groove in a state of contact. Therefore, there is a possibility that the contact force and restraining force of the needle tip protector against the puncture needle may become too large, and the operability may be hindered due to frictional resistance or catching, particularly during withdrawal operation of the puncture needle.

Therefore, according to the needle tip protector structured following the thirteenth preferred embodiment, a distal end edge of at least one of the first side plate and the second side plate includes a notch extending in the plate width direction. With this configuration, in the state of being mounted onto the puncture needle, wobbling of the needle tip protector or the like is suppressed, so that stability can be improved by the guiding action during movement to the needle tip side. In particular, with the groove extending in the needle axis direction as described in Patent Document 1, there are concerns about frictional resistance during movement of the puncture needle, catching against the puncture needle during tilting and the like, but such concerns are avoided by providing the notch extending in the plate width direction of the side plate.

Regarding the structures described in the preceding first through thirteenth preferred embodiments, two or more structures selected as appropriate may be employed in combination.

The needle tip protector according to the present invention can also be applied to a needle assembly with a hemostasis valve in which a lumen of an outer needle hub is blocked by the hemostasis valve after a puncture needle is removed.

### KEYS TO SYMBOLS

- 10: needle tip protector (first practical embodiment)
- 12: needle assembly
- 14: outer needle unit
- 16: inner needle unit
- 18: outer needle
- 20: outer needle hub
- 22: caulking pin
- 23: screw part
- 24: locking shoulder part
- 26: inner needle (puncture needle)
- 28: inner needle hub
- 30: needle tip
- 32: blade surface
- 34: connecting part
- 36: filter cap
- 38: filter
- 40: bottom part
- 42: first side plate
- 44: second side plate
- 46: insertion hole
- 48: first main body
- 50: first needle-tip protection part (needle-tip guarding part)
- 52: first distal-end protection piece
- 54: first return piece
- 56: lateral guarding piece
- 58: first stepped part
- 60: first notch (notch)
- 62: first lateral cover piece (lateral cover piece)
- 64: first concave part (concave part)
- 66: first protrusion piece (protrusion piece)
- 68: second main body
- 70: second needle-tip protection part (needle-tip guarding part)
- 72: second distal-end protection piece
- 74: second return piece
- 76: second stepped part
- 78: second notch (notch)
- 80: second lateral cover piece (lateral cover piece)
- 82: second concave part (concave part)
- 84: second protrusion piece (protrusion piece)
- 86: molding space
- 88: large-diameter part
- 90: needle tip protector (second practical embodiment)
- 92: needle assembly
- 94: first side plate
- 96: second side plate
- 97: thin-walled part (stress transmission suppressing part)
- 98: first needle-tip protection part (needle-tip guarding part)
- 100: first needle-tip cover part (needle-tip cover part)
- 102: first needle-tip stopper part (needle-tip stopper part)
- 104: second needle-tip protection part (needle-tip guarding part)
- 106: second needle-tip cover part (needle-tip cover part)
- 108: second needle-tip stopper part (needle-tip stopper part)
- 110: thin-walled part
- 120: needle tip protector (third practical embodiment)
- 122: needle assembly
- 124: bottom part
- 126: convex part
- 128: first relief part (relief part)
- 130: second relief part (relief part)
- 132: lateral guarding piece
- 134: first regulating protrusion (regulating protrusion)
- 136: second regulating protrusion (regulating protrusion)
- 138: outer needle hub
- 140: expanded housing part
- 142: locking protrusion

## Claims

1. A needle tip protector configured to prevent exposure of a needle tip by being attached to a puncture needle and moved to a needle tip side, comprising:
a bottom part located on a needle base side of the puncture needle;
a first side plate and a second side plate extending from the bottom part to the needle tip side of the puncture needle, the first side plate and the second side plate being arranged facing each other while sandwiching the puncture needle from both sides; and
lateral cover pieces provided to respective widthwise edges of the first side plate and the second side plate and protruding inward in a direction of facing, the respective widthwise edges being opposite to each other in a plate width direction, wherein
with the needle tip protector attached to the puncture needle, the puncture needle is located between the respective lateral cover pieces of the first side plate and the second side plate.

2. The needle tip protector according to claim 1, wherein respective centers of the first side plate and the second side plate in the plate width direction are mutually shifted to sides opposite to the corresponding lateral cover pieces.

3. The needle tip protector according to claim 1 or 2, wherein
protrusion pieces are provided to respective other widthwise edges of the first side plate and the second side plate and protrude inward in the direction of facing, the respective other widthwise edges being located opposite to the lateral cover pieces in the plate width direction, and
the protrusion pieces are located on outer sides of the corresponding lateral cover pieces.

4. The needle tip protector according to any one of claims 1-3, wherein
the bottom part has a plate shape including an insertion hole for the puncture needle, and the first side plate and the second side plate are integrally formed with the bottom part such that the first side plate and the second side plate rise from respective edges of the bottom part, and
the lateral cover pieces are provided not less than 2.5 mm apart from proximal ends of the first side plate and the second side plate such that a molding space opened to both sides in the plate width direction is provided between facing surfaces of proximal end portions of the first side plate and the second side plate ahead of the bottom part.

5. The needle tip protector according to claim 1 or 2, wherein in a needle-tip protection state where the first side plate and the second side plate approach each other and the exposure of the needle tip is prevented, protruding distal ends of the lateral cover pieces of the first side plate and the second side plate are respectively pressed against the second side plate and the first side plate.

6. A needle tip protector configured to prevent exposure of a needle tip by being attached to a puncture needle and moved to a needle tip side, comprising:
a bottom part located on a needle base side of the puncture needle; and
a first side plate and a second side plate extending from the bottom part to the needle tip side of the puncture needle, the first side plate and the second side plate being arranged facing each other while sandwiching the puncture needle from both sides, wherein
at least one of the first side plate and the second side plate is configured such that a center in a plate width direction is shifted from a needle axis of the puncture needle in the plate width direction, and includes a lateral cover piece at a widthwise edge on a side approaching the puncture needle, the lateral cover piece protruding inward in a direction of facing of the first side plate and the second side plate.

7. A needle tip protector configured to prevent exposure of a needle tip by being attached to a puncture needle and moved to a needle tip side, comprising:
a bottom part located on a needle base side of the puncture needle; and
a first side plate and a second side plate extending from the bottom part to the needle tip side of the puncture needle, wherein
at least one of the first side plate and the second side plate includes:
a notch-shaped concave part opening at a side end in a plate width direction; and
a lateral cover piece protruding inward from the notch-shaped concave part and being located on a lateral side of the puncture needle.

8. A needle tip protector configured to prevent exposure of a needle tip by being attached to a puncture needle and moved to a needle tip side, comprising:
a bottom part located on a needle base side of the puncture needle; and
a first side plate and a second side plate extending from the bottom part to the needle tip side of the puncture needle, wherein
a distal end portion of at least one of the first side plate and the second side plate includes a needle-tip cover part protruding toward the puncture needle, and
a protruding distal end of the needle-tip cover part includes a needle-tip stopper part protruding to the needle base side.

9. A needle tip protector configured to prevent exposure of a needle tip by being attached to a puncture needle and moved to a needle tip side, comprising:
a bottom part located on a needle base side of the puncture needle;
a first side plate and a second side plate extending from the bottom part to the needle tip side of the puncture needle, the first side plate and the second side plate being arranged facing each other while sandwiching the puncture needle from both sides; and
a lateral regulating piece extending between facing surfaces of the first side plate and the second side plate, wherein
the lateral regulating piece includes a regulating protrusion protruding toward the puncture needle.

10. The needle tip protector according to claim 9, wherein the regulating protrusion is provided at an end on the needle tip side of the lateral regulating piece.

11. The needle tip protector according to claim 9 or 10, wherein the lateral regulating piece protrudes inward from the first side plate or the second side plate in a direction of facing of the first side plate and the second side plate.

12. A press-formed needle tip protector configured to prevent exposure of a needle tip by being attached to a puncture needle and moved to a needle tip side, comprising:
a bottom part located on a needle base side of the puncture needle; and
a first side plate and a second side plate bent and extending from the bottom part to the needle tip side of the puncture needle, the first side plate and the second side plate being arranged facing each other, wherein
the bottom part has a plate shape including an insertion hole for the puncture needle, and the first side plate and the second side plate are integrally formed with the bottom part such that the first side plate and the second side plate rise from respective edges of the bottom part, and
the bottom part includes a convex part protruding outward in a plate width direction of the first side plate and the second side plate.

13. A needle tip protector configured to prevent exposure of a needle tip by being attached to a puncture needle and moved to a needle tip side, comprising:
a bottom part located on a needle base side of the puncture needle; and
a first side plate and a second side plate extending from the bottom part to the needle tip side of the puncture needle, the first side plate and the second side plate being arranged facing each other while sandwiching the puncture needle from both sides, and distal end portions of the first side plate and the second side plate each forming a needle-tip guarding part configured to cover the needle tip by approaching each other, wherein
the needle-tip guarding part protrudes outward at a proximal end that is the needle base side, and
the proximal end of the needle-tip guarding part includes notch-shaped relief parts on respective sides in a plate width direction.

14. A needle tip protector configured to prevent exposure of a needle tip by being attached to a puncture needle and moved to a needle tip side, comprising:
a bottom part located on a needle base side of the puncture needle;
a first side plate and a second side plate extending from the bottom part to the needle tip side of the puncture needle, the first side plate and the second side plate being arranged facing each other, wherein
the bottom part has a plate shape including an insertion hole for the puncture needle, and the first side plate and the second side plate are integrally formed with the bottom part such that the first side plate and the second side plate rise from respective edges of the bottom part, and
the bottom part includes a thin-walled stress transmission suppressing part surrounding the insertion hole.
